# EUROPEAN PATENT SPECIFICATION

(11) **EP 1 622 888 B1**
(45) Date of publication and mention of the grant of the patent: **08.07.2009**
(21) Application number: 04751581.2
(22) Date of filing: 05.05.2004
(51) Int. Cl.: C07D 311/92, C07D 311/78, C07D 493/04

(54) **BENZO-, NAPHTHO- AND PHENANTHROCHROMENES SUBSTITUTED WITH AN ARYLAMINE GROUP WITH PHOTOCHROMIC PROPERTIES**
BENZO-, NAPHTHO- UND PHENANTHROCHROMENE SUBSTITUIERT MIT EINER ARYLAMINE GRUPPE MIT PHOTOCHROMEN EIGENSCHAFTEN
BENZO, NAPHTO ET PHENANTHROCHROMENES SUBSTITUES PAR UN GROUPE ARYLAMINE POSSEDANT DES PROPRIETES PHOTOCHROMIQUES

(30) Priority: 05.05.2003 FR 0305437
(43) Date of publication of application: 08.02.2006
(73) Proprietor: Corning Incorporated, Corning NY 14831 (US)
(72) Inventor: BREMAND, Rodrigue, 33640 Ayguemorte-les-Graves (FR); BREYNE, Oliver, F-69004 Lyon (FR); CHAN, You-Ping, F-69008 Lyon (FR)
(74) Representative: Behnisch, Werner
(86) International application number: PCT/US2004/014240
(87) International publication number: WO 2004/099172

(56) References cited:
- WO-A-00/15628
- GABBUTT C D ET AL: "Synthesis and photochromic properties of some fluorine-containing naphthopyrans" DYES AND PIGMENTS, ELSEVIER APPLIED SCIENCE PUBLISHERS. BARKING, GB, vol. 54, no. 1, July 2002 (2002-07), pages 79-93, XP004370491 ISSN: 0143-7208

## Description

### FIELD OF THE INVENTION

The present invention relates to novel benzo-, naphtho- or phenanthrochromene-type compounds which have, in particular, photochromic properties. The invention also relates to photochromic compositions and photochromic ophthalmic articles (lenses for example) which contain said naphthopyrans. The invention also covers the preparation of these novel compounds.

### BACKGROUND OF THE INVENTION

The photochromic compounds are capable of changing color under the influence of a poly- or mono-chromatic light (UV for example) and of returning to their initial color when the luminous irradiation ceases, or under the influence of temperature and/or a poly- or mono-chromatic light different from the first.

The photochromic compounds find applications in various fields, *e.g*. for the manufacture of ophthalmic lenses, contact lenses, solar protection glasses, filters, camera optics or photographic apparatus optics or other optical devices and observation devices, glazing, decorative objects, bill elements or even for information storage by optical inscription (coding).

In the field of ophthalmic optics, and in particular the spectacles trade, a photochromic lens which comprises one or more photochromic compounds must have:
- a high transmission in the absence of ultraviolets,
- a low transmission (high colorability) under solar irradiation,
- adapted coloration and discoloration kinetics,
- a tint acceptable to the consumer (grey or brown preferably) with preferably a maintenance of the chosen tint during the coloration and the discoloration of the lens,
- a maintenance of the performances, the properties, within a temperature range of 0-40°C,
- a significant durability, since these objectives sought after are sophisticated corrective lenses and therefore expensive.

These lens characteristics are in fact determined by the active photochomic compounds which said lens contains; compounds which must furthermore be perfectly compatible with the organic or inorganic, even hybrid support which constitutes said lens.

Moreover, it is to be noted that obtaining a grey or brown tint may necessitate the use of at least two photochromes of different colors, *i.e*. having distinct maximal absorption wavelengths in the visible. This combination further inposes other requirements of the photochromic compounds. In particular, the coloration and discoloration kinetics of the (two or more) combined active photochromic compounds must be essentially identical. The same applies for their stability with time and also for their compatibility with a plastic, inorganic or hybrid inorganic-organic support.

Amongst the numerous photochromic compounds described in the prior art, compounds may be cited which are described in the patents or patent applications: US-A-3,567,605, US-A-3,627,690, US-A-4,826,977, US-A-5,200,116, US-A-5,238,981, US-A-5,411,679, US-A-5,429,744, US-A-5,451,344, US-A-5,458,814, US-A-5,651,923, US-A-5,645,767, US-A-5,698,141, US-A-5,783,116, US-A-5,888,432, US-A-6,096,246, US-B-6,023,729, US-B-6,207,084, US-B-6,210,608, US-B-6,291,561, WO-A-95 05382, FR-A-2,718,447, WO-A-96 14596, WO-A-97 21698, WO-A-00 15628, WO-A- 32661, WO-A-01 36424, which are of the reduced formulae below:

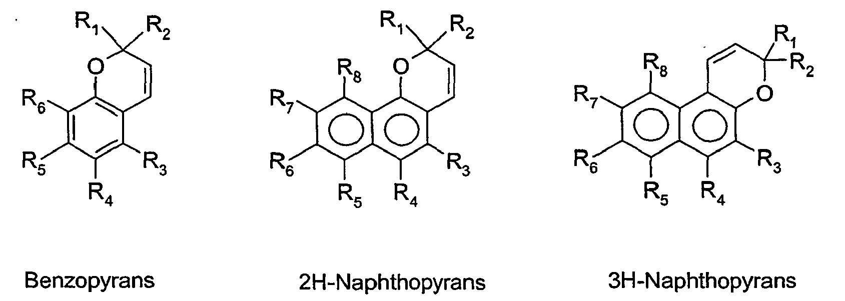

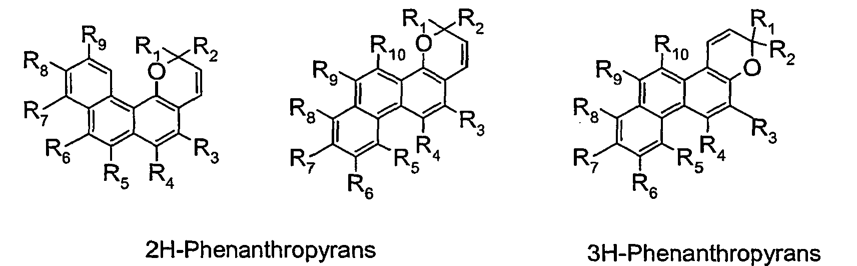

When said compounds are substituted, they are obviously done so in order that the pyran ring of their formula can open.

Thus, for the 3H-naphthopyrans, R₈ must equal H and for the 3H-phenanthropyrans, R₁₀ must similarly be H.

The patent application WO-A-01 36406 describes 3H-naphthopyrans having a dihydronaphthalene group linked to carbons 5 and 6 of the naphtho skeleton:

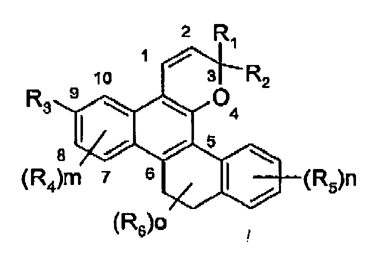

These compounds claim to satisfy the specifications defined *supra.* In reality, if these compounds really do have one or more of the basic properties sought after, such as a high transmission in the absence of ultraviolets and a high colorability under solar irradiation, none of the compounds described hitherto have the complete combination of the properties sought after which are necessary for the production of satisfactory articles. In particular, not one of these compounds is intrinsically grey or brown, and the necessity of using an additional photochrome in order to obtain one of these two tints does subsist.

In this context, it is useful to be able to have photochromes at one's disposal for each coloration, thus enabling a fine adjustment of the tint. The presence of donor groups in the para position of a phenyl group in R₁ or in R₂ (in position 2 of the pyran ring) enables a more or less significant bathochromic shift of the absorption bands of the photochrome. However, the shift obtained by substituting an alkoxy group by an amino group is very high (30-40 nm minimum). Groups enabling an intermediate shift to be obtained are thus necessary.

Japanese patent application JP 2001-114775 describes compounds the R₁ group of which is a phenyl substituted in para with an amine substituted with electron attracting groups. The wavelengths of the absorption bands are thus clearly lowered (-15 to -30 nm) with respect to the Comparative Example (morpholino group). In contrast, the discoloration kinetics are strongly penalised (data not mentioned).

It is to the credit of the inventors for having been interested in this type of derivative as a basis for the development of novel photochromes and for having proposed a novel family of molecules having particularly advantageous photochromic properties.

### DESCRIPTION OF THE INVETION

Thus, according to a first of its aspects, the present invention relates to benzo-, naphtho- and phenanthrochromenes of formulae (Ia), (Ib), (Ic) and (Id), below: 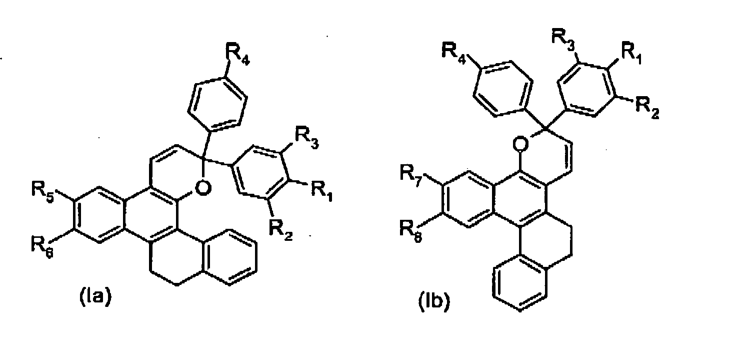 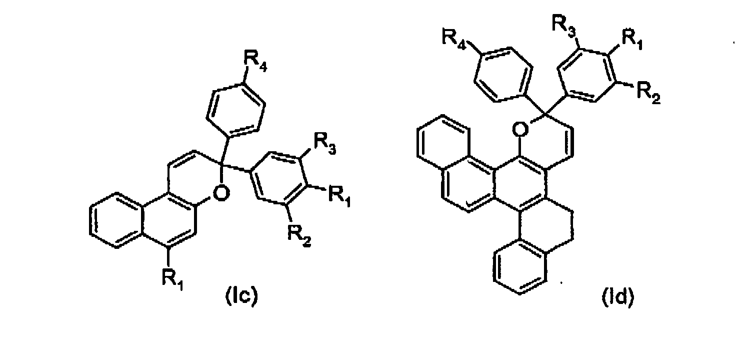 in which:
- R₁ represents a: 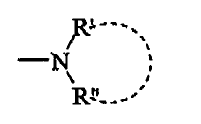 group, R' and R", which are identical or different, independently representing a linear or branched alkyl group which comprises 1 to 6 carbon atoms, a phenyl group which is optionally substituted with at least one linear or branched alkyl group which comprises 1 to 6 carbon atoms, or representing, together with the nitrogen atom to which they are bound, a 5- to 7-membered ring which optionally comprises at least one other heteroatom selected from oxygen, sulphur and nitrogen, said nitrogen being optionally substituted with an R"' group, which is a linear or branched alkyl group having 1 to 6 carbon atoms; the two R₁ substituents of formula (Ic) being, independently, identical or different,
- one of R₂ and R₃ represents:
   - a halogen, and notably fluorine, chlorine or bromine,
   - a linear or branched alkyl group which comprises 1 to 12 carbon atoms (advantageously 1, to 6 carbon atoms),
   - a cycloalkyl group which comprises 3 to 12 carbon atoms (advantageously 3 to 6 carbon atoms),
   - a linear or branched alkoxy group which comprises 1 to 12 carbon atoms (advantageously 1 to 6 carbon atoms), or
   - a phenyl or benzyl group, which is optionally substituted,
   and the other of R₂ and R₃ represents:
   - a hydrogen or
   - a linear or branched alkyl group which comprises 1 to 6 carbon atoms;
- R₄ represents:
   - a hydrogen,
   - a linear or branched alkyl group which comprises 1 to 12 carbon atoms (advantageously 1 to 6 carbon atoms),
   - a cycloalkyl group which comprises 3 to 12 carbon atoms (advantageously 3 to 6 carbon atoms),
   - a linear or branched alkoxy group which comprises 1 to 12 carbon atoms (advantageously 1 to 6 carbon atoms),
   - a phenyl or benzyl group, which is optionally substituted;

The person skilled in the art will obviously have understood that the branched alkyl and alkoxy groups as defined above comprise a numbed of carbons which is sufficient for them to be able to be branched (more than 3 carbon atoms).

The person skilled in the art will furthermore have understood that the invention is situated around the structure of the substituents in position 2 of the pyran ring (phenyl substituents) and that the invention is available for a very large context of benzo-, naphtho- and phenanthropyrans. The nature of the benzo-, naphtho-and phenanthrogroup is critical only with regard to one point: it must not comprise any substituent(s) which prevent the opening of the pyran ring (said opening conferring the photochromic properties to the compounds in question). It will be noted *inter alia* that said group can indeed contain substituents bearing unsaturation(s), such unsaturations enabling the polymerisation and/or the cross-linking and/or the grafting of the compound in question.

As regards the values of R₂ or R₃ and R₄ = substituted phenyl or benzyl, an indication can be made in a totally non-limiting way that the substituents in question can notably be selected from linear or branched alkyl groups which comprise 1 to 6 carbon atoms, linear or branched alkoxy groups which comprise 1 to 6 carbon atoms, and halogen atoms (more particularly chlorine, fluorine and bromine). Other substituents can be incorporated and can contain unsaturations, enabling the polymerisation and/or the cross-linking and/or the grafting of the compound in question.
- R₅ represents:
   - a hydroxy group,
   - a linear or branched alkyl group which comprises 1 to 6 carbon atoms,
   - a linear or branched alkoxy group which comprises 1 to 6 carbon atoms,
   - an R₁ group, as defined above, *i.e.* a: 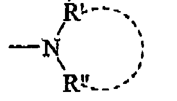 group,
- R₆ represents:
   - a halogen, and notably fluorine, chlorine or bromine,
   - a hydroxy,
   - a linear or branched alkyl group which comprises 1 to 6 carbon atoms,
   - a linear or branched alkoxy group which comprises 1 to 6 carbon atoms,
   - an aryl or heteroaryl group which comprises, in its basic structure, 6 to 24 carbon atoms or 4 to 24 carbon atoms respectively and at least one heteroatom selected from sulphur, oxygen and nitrogen; said basic structure being optionally substituted with at least one substituent selected from the whole of the substituents given below:
      + a halogen, and notably fluorine, chlorine and bromine,
      + a hydroxy,
      + a linear or branched alkyl group which comprises 1 to 12 carbon atoms (advantageously 1 to 6 carbon atoms),
      + a linear or branched alkoxy group which comprises 1 to 12 carbon atoms (advantageously 1 to 6 carbon atoms),
      + a haloalkyl or haloalkoxy group corresponding to the (C₁-C₁₂) alkyl or alkoxy groups above, respectively, which are substituted with at least one halogen atom, and notably a fluoroalkyl group of this type,
      + a phenoxy or naphthoxy group which is optionally substituted witch at least one linear or branched alkyl or alkoxy group which comprises 1 to 12 carbon atoms,
      + an -NH₂ group,
      + an -NHR group, R representing a linear or branched alkyl group which comprises 1 to 6 carbon atoms, or a phenyl group which is optionally substituted with at least one linear or branched alkyl group which comprises 1 to 6 carbon atoms,
      + an R₁ group, as defined above, *i.e.*, a 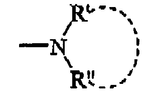 group,
   or
   R₅ and R₆ together form an aromatic or non-aromatic cyclic group having one or two 5- to 7-membered annelated rings and optionally comprising at least one heteroatom selected from oxygen, sulphur, and nitrogen; said annelated rings optionally comprising at least one R₄ substituent, as defined above, which is different from hydrogen;
- R₇ and R₈, which are identical or different, independently represent:
   - a hydrogen,
   - a halogen, and notably fluorine, chlorine or bromine,
   - a linear or branched alkyl group which comprises 1 to 12 carbon atoms (advantageously 1 to 6 carbon atoms),
   - a cycloalkyl group which comprises 3 to 12 carbon atoms (advantageously 3 to 6 carbon atoms),
   - a linear or branched alkoxy group which comprises 1 to 12 carbon atoms (advantageously 1 to 6 carbon atoms),
   - a haloalkyl, halocycloalkyl, or haloalkoxy group corresponding to the alkyl, cycloalkyl or alkoxy groups above, respectively, which are substituted with at least one halogen atom, notably selected from fluorine, chlorine and bromine,
   - an aryl or heteroaryl group, having the same definition as the aryl or heteroaryl, defined with respect to R₆,
   - an aralkyl or heteroaralkyl group, the alkyl group, which is linear or branched, comprising 1 to 4 carbon atoms, and the aryl and heteroaryl groups having the same definitions as those given above,
   - a phenoxy or naphthoxy group which is optionally substituted with at least one linear or branched alkyl or alkoxy group which comprises 1 to 12 carbon atoms,
   - an amine, amide, carbonate or urea group: -NH₂, -NHR, -NHCOR, -NHCOOR, NR'COR, -NHCONHR, -CONH₂, -CONHR, 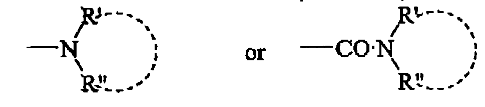 R, R', R" having their respective definitions given above for the amine substituents of R₆ where R₆ is an aryl or heteroaryl,
   - an -OCOR₁₁ or -COOR₁₁ group, R₁₁, representing a straight or branched alkyl group comprising 1 to 6 carbon atoms, or a cycloalkyl group comprising 3 to 6 carbon atoms, or a phenyl group, optionally substituted with at least one substituent selected from the whole of the substituents listed above for R₆ where R₆ is an aryl or heteroaryl,
      or
      R₇ and R₈ together form an aromatic or non-aromatic cyclic group, optionally comprising at least one heteroatom selected from the group consisting of: oxygen, sulphur, and nitrogen, and having one or two 5- to 7-membered annelated rings; said annelated rings being optionally substituted with at least one substituent selected from the whole of the substituents listed above for R₆ where R₆ is an aryl or heteroaryl.

The compounds of formula (Ia) and (Ic) are 3H-naphthopyrans, those of formula (Ib) are 2H-naphthopyrans, while those of formula (Id) are 2H-phenanthropyrans.

Amongst the compounds of the invention, those of formula (I), (Ia), (Ib), (Ic) and (Id) are preferred, in which:
- one of R₂ and R₃ represents:
   a halogen, a linear or branched alkyl group which comprises 1 to 6 carbon atoms, an optionally substituted phenyl or benzyl group,
   and the other of R₂ and R₃ represents:
   a hydrogen, or a linear or branched alkyl group which comprises 1 to 6 carbon atoms;
   and/or, advantageously and
- R₄ represents:
   - a hydrogen,
   - a linear or branched alkyl group which comprises 1 to 12 carbon atoms (advantageously 1 to 6 carbon atoms),
   - a linear or branched alkoxy group which comprises 1 to 12 carbon atoms (advantageously 1 to 6 carbon atoms).

According to a second of its aspects, the present invention relates to a method of preparing compounds of formulae (Ia) to (Id), characterised in that it comprises a condensation of:
- an intermediate product of formula (II) given below: 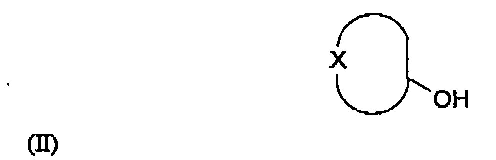 in which the 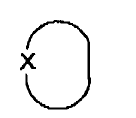 group is
   one of the following groups 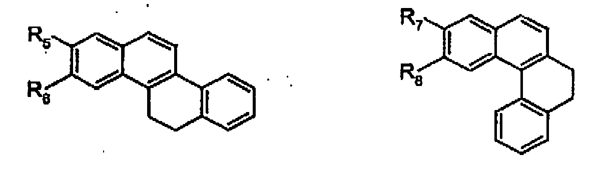 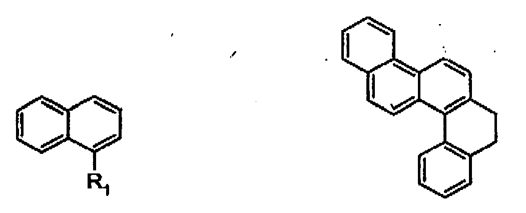 in which
- R₁, R₅, R₆, R₇ and R₈ are as defined above with reference to formulae (Ia) to (Id).
- a derivative of propargylic alcohol, having formula (III) below: 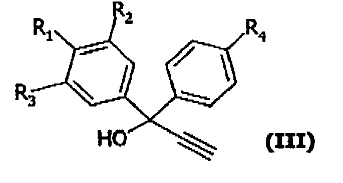 in which R₁, R₂, R₃ and R₄ are as defined *supra* with reference to formulae (Ia) to (Id);
   the condensation of (II) and (III) being carried out advantageously in the presence of a catalyst, this catalyst being preferably selected from the group consisting of para-toluenesulphonic acid, dodecylsulphonic acid, camphorsulphonic acid, or bromoacetic acid;
   or with
- an aldehyde derivative, having formula (III') bellow: 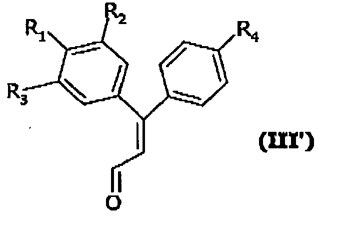 in which R₁, R₂, R₃ and R₄ are as defined *supra* with reference to formulae (Ia) to (Id);
   the condensation of (II) and (III') being carried out, advantageously, in the presence of a metallic complex, preferably a complex of titanium, particularly titanium (IV) ethoxide.

In practice, the condensation reaction between compounds (II) and (III) or (III') can take place in solvents such as toluene, xylene or tetrahydrofuran, to which appropriate catalysts are optionally added. For more details on the condensation of compounds (II) and (III'), reference maybe made to the EP-A-0 562 915 patent application.

The compounds of formula (III) are known to the person skilled in the art and are obtained from the corresponding ketone according to a method described notably in the WO-A-96 14596 patent application. The ketone is itself commercial or is prepared according to known methods such as the Friedel Crafts reaction (cf. WO-A-96 14596 and cited references).

Aldehydes (III'), which are derivatives of (III), are obtained by rearrangement in an acid medium *(cf.* J. Org. Chem., 1977, 42, 3403).

The compounds of formula (II) are phenols, naphthols or phenanthrols which are optionally substituted, and the synthetic routes to which are described in the prior art.

According to a third of its aspects, an object of the invention is (co)polymers which are obtainable by polymerising and/or cross-linking and/or grafting at least one compound (Ia) to (Id) as defined above. The compounds (Ia) to (Id) according to the invention can be *per se* (co)monomers and/or be comprised in (co)polymerisable (co)monomers. The (co)polymers thus obtained (which are optionally cross-linked and/or grafted) can constitute photochromic matrices such as those presented *infra.*

According to a fourth of its aspects, the present invention relates to the use of said compounds of formulae (Ia) to (Id) of the invention as photochromic agents. Another object of the invention is, therefore:
- firstly, novel photochromic compounds which are constituted by the chromenes of formulae (Ia) to (Id), as defined above, taken alone or in a mixture of themselves and/or with at least one other photochromic compound of another type and/or with at least one non-photochromic coloring agent;
- secondly, novel photochromic compositions which comprise at least one compound (Ia) to (Id) as defined above, or at least one (co)polymer of the above type. Such photochromic compositions can contain at least one other photochromic compound, of another type and/or at least one non-photochromic coloring agent and/or at least one stabilising agent. These photochromic compounds of another type, non-photochromic coloring agents, and stabilising agents, are prior art products known to the person skilled in the art.

Within the context of the present invention, combinations of photochromic compounds of the invention and/or combinations of photochromic compounds of the invention and photochromic compounds of another type according to the prior art are particularly recommended; such combinations being interesting in that they are suitable for generating grey or brown tints, which are desired by the public in applications such as ophthalmic spectacles or solar spectacles. These additional photochromic compounds can be those known to the person skilled in the art and described in the literature, *e.g*. chromenes (US-A-3,567,605, US-A-5,238,981, WO-A-94 22850, EP-A-0 562 915), spiropyrans or naphthospiropyrans (US-A-5,238,981) and spiroxazines (Crano et al., " Applied Photochromic Polymer Systems ", Ed. Blackie & Son Ltd, 1992, chapter 2).

The compositions according to the invention can in fact comprise, in addition to the photochromic compounds:
- non-photochromic coloring agents which enable adjusting the tint,
- and/or one or more stabilising agents, such as an anti-oxidising agent for example,
- and/or one or more anti-UV,
- and/or one or more anti-radicals,
- and/or one or more photochimic excited state deactivators.
These additives can notably enable improving the durability of said compositions.

The compounds of the invention envisaged within the context of their photochromic applications can be used in liquid solution. Thus, a photochromic solution can be obtained by dissolving at least one of said compounds in an organic solvent such as toluene, dichloromethane, tetrahydrofuran or ethanol. The solutions obtained are in general colorless and transparent. When exposed to sunlight, they develop a strong coloration and regain the colorless state when they are placed in an area of less exposure to the sun's rays or, in other words, when they are no longer subjected to UV. In general, a very low concentration of product (of the order of 0.01 to 5% by weight) is sufficient to obtain an intense coloration.

The compounds according to the invention are furthermore compatible with support matrices of organic polymer or of inorganic material (even in a hybrid inorganic-organic material).

Also, within the context of the fourth aspect of the invention in relation to the photochromic applications, an object of the invention is a photochromic composition selected from:
- photochromic solutions, and
- photochromic matrices.

The most interesting applications of the compounds of the invention are in fact those in which the photochrome is dispersed uniformly within a matrix formed by a polymer and/or copolymer and/or mixture of (co)polymers. Such a matrix can constitute, in accordance with a first variant, a photochromic substrate *per se* (in its volume), and according to a second variant, a photochromic coating (intended to be incorporated on the surface of an element).

Following the example of their behaviour in solution, the compounds (Ia) to (Id), included in a polymer matrix, are colorless or slightly colored in the initial state and rapidly develop an intense coloration under a UV light (365 nm) or under a light source of the solar type. Finally, they regain their initial coloration once the irradiation ceases.

The methods of implementation which can be envisaged in order to obtain such a matrix are very varied. Amongst those known to the person skilled in the art, the diffusion in the (co)polymer, from a suspension or solution of the photochrome, in a silicone oil, in an aliphatic or aromatic hydrocarbon, or in a glycol, or from another polymer matrix, can be cited for example. The diffusion is commonly carried out at a temperature of 50 to 200°C for a period of time of 15 minutes to several hours, according to the nature of the polymer matrix. Another implementation technique consists in mixing the photochrome in a formulation of polymerisable materials, depositing this mixture on a surface or in a mould, and then carrying out the copolymerisation. These implementation techniques, and others, are described in the article by Crano et al. " Spiroxazines and their use in photochromic lenses " published in Applied Photochromic Polymer Systems, Ed. Blackie and Son Ltd - 1992.

The following products may be mentioned as examples of preferred polymer materials for forming matrices which are useful in optical applications of the photochromic compounds according to the invention:
- those obtained from alkyl, cycloalkyl, (poly or oligo)ethylene glycol, aryl or arylalkyl mono-, di- tri- or tetraacrylates or mono-, di-, tri- or tetramethacrylates, which are optionally halogenated or which comprise at least one ether and/or ester and/or carbonate and/or carbamate and/or thiocarbamate and/or urea and/or amide group,
- polystyrene, polyether, polyester, polycarbonate (e.g. bisphenol-A polycarbonate, diallyl diethylene glycol polycarbonate), polycarbamate, polyepoxy, polyurea, polyurethane, polythiourethane, polysiloxane, polyacrylonitrile, polyamide, aliphatic or aromatic polyester, vinylic polymers, cellulose acetate, cellulose triacetate, cellulose acetate-propionate or polyvinylbutyral,
- those obtained from difunctional monomers having the formula below: 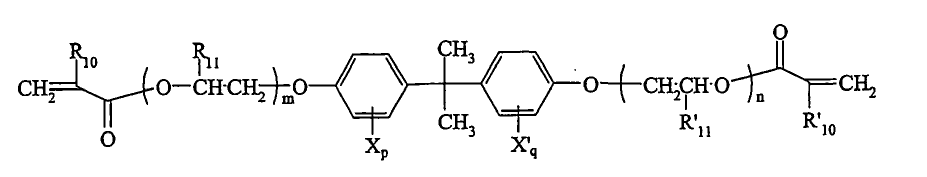 in which:
   Δ R₁₀, R'₁₀, R₁₁ and R'₁₁ are identical or different and represent independently a hydrogen or a methyl group;
   Δ m and n are, independently, integers between 0 and 4 (inclusive); and are advantageously independently equal to 1 or 2;
   Δ X and X', which are identical or different, are a halogen and represent, preferably, a chlorine and/or a bromine;
   Δ p and q are, independently, integers between 0 and 4 (inclusive);
      - copolymers of at least two types of copolymerisable monomers selected from the precursor monomers of the polymers listed *supra,* and preferably those belonging to the groups comprising: (meth)acrylic monomers, vinylic monomers, allylic monomers, and mixtures thereof.

In a particularly preferred manner, the photochromes of the invention are used:
- with resins which have a nanobiphasic structure and which are obtainable by copolymerising at least two specific, different difunctional monomers. Such resins have been described in the patent application WO-A-98 50443;
- with resins obtainable by co-polymerisation of at least one monofunctional monomer and at least one difunctional monomer, as described in WO-A-01 92 372;
- with resins obtainable by copolymerising a composition containing at least one monofunctional monomer, at least one other monomer selected from monofunctional monomers and alkenic difunctional monomers and an effective amount of at least one acid of basic additive, as described in WO-A-02 06364; and
- with resins based on linear polymers of polyurethane or polyurethane-urea type, which comprise at least one unsaturation in their chain. Such resins are described in the French patent application FR 02 04778, which is hitherto not published.

The amount ofphotochrome used in the (co)polymer matrix depends upon the degree of darkening desired. Usually, between 0.001 and 20% by weight of it is used.

Still according to the fourth of its aspects in relation to the applications of the compounds (I) as photochromes, another object of the present invention is photochromic articles comprising a photochromic composition as described above, notably a photochromic matrix in its volume.

In practice, the photochromic articles which are more particularly covered by the present invention are ophthalmic or solar lenses, glazing (windows for buildings, for locomotion engines, automobile vehicles), optical devices, decorative articles, solar protection articles, information storage, ...

The present invention is illustrated by the Examples which follow, of synthesis and of photochromic validation, of compounds of the invention. Said compounds of the invention are compared to a prior art compounds C1 to C5.

### EXAMPLES

EXAMPLE 1: SYNTHESIS oF COMPOUND (1) 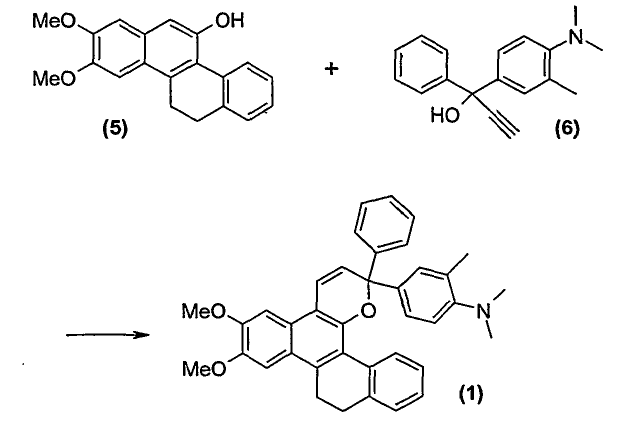 200 mg of camphorsulphonic acid are added to a solution of 350 mg of naphthol (5) and 320 mg of propargylic acid (6) in 15 ml of toluene. Stirring at 60 °C is effected for 3 hours, and the reaction mixture is then purified by filtration on silica. The solid obtained is recrystallised to give 95 mg of crystals which are pure by ¹H NMR.

EXAMPLE 2: SYNTHESIS oF COMPOUND (2) 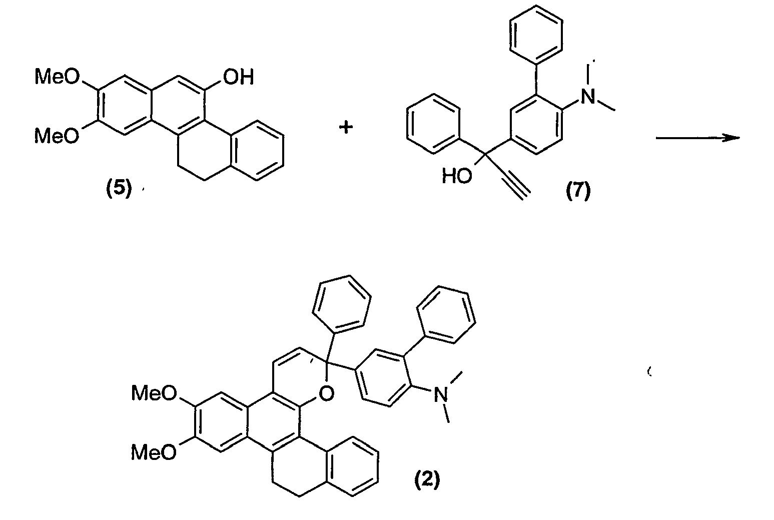 18 mg of camphorsulphonic acid are added to a solution of 370 mg of naphthol (5) and 450 mg of propargylic alcohol (7) in 15 ml of toluene. Stirring is effected at 50 °C for 2 hours, and the reaction mixture is then purified by filtration on silica. The solid obtained is recrystallised to give 320 mg of crystals which are pure by ¹H NMR.

EXAMPLE 3: SYNTHESIS oF COMPOUND (3) 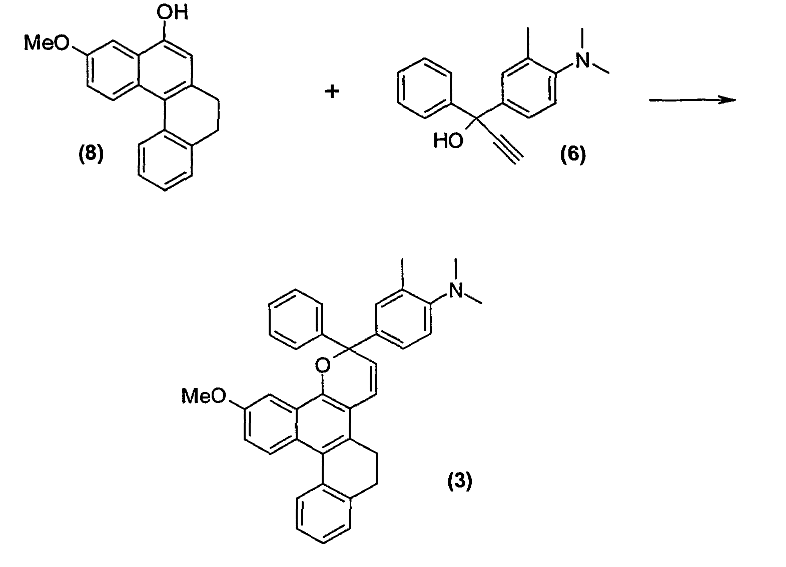 84 mg of camphorsulphonic acid are added to a solution of 500 mg of naphthol (8) and 528 mg of propargylic alcohol (6) in 10 ml of toluene. Stirring is effected at 60°C for 6h, and the reaction mixture is then purified by filtration on silica. The solid obtained is recrystallised to give 19 mg of crystals which are pure by ¹H NMR.

EXAMPLE 4: SYNTHESIS OF COMPOUND (4) 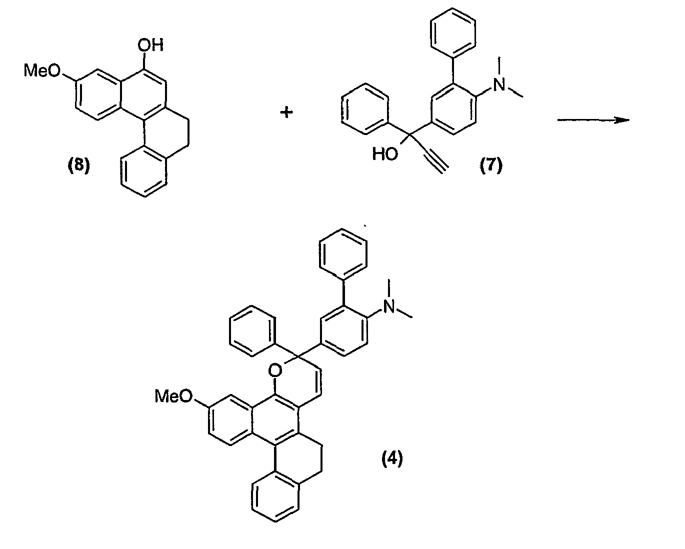 585 mg of camphorsulphonic acid are added to a solution of 660 mg of naphthol (8) and 860 mg propargylic alcohol (7) in 15 ml of toluene. Stirring is effected at 50 °C for 3 hours, and the reaction mixture is then purified by filtration on silica. The solid obtained is recrystallised to give 7 mg of crystals which are pure by ¹H NMR.

EXAMPLE 5: SYNTHESIS OF COMPOUND (5) 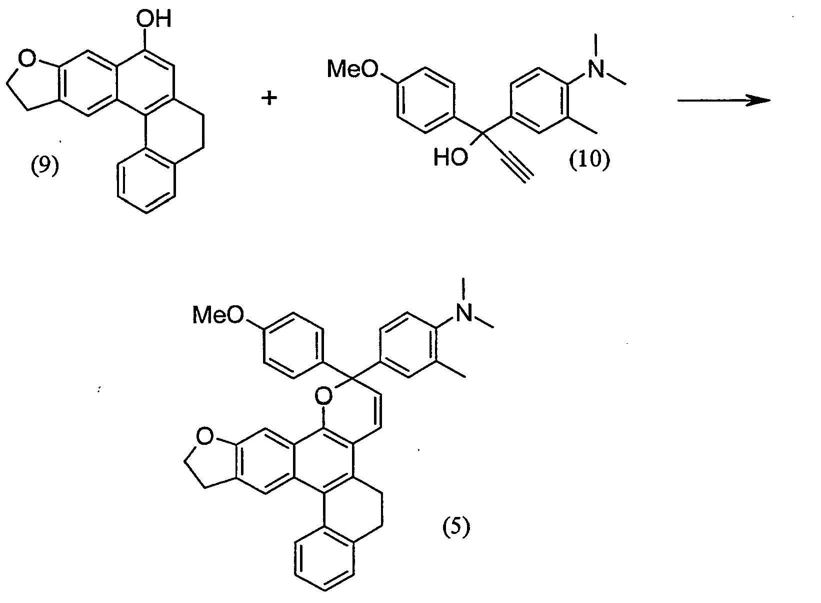 511 mg of camphorsulphonic acid are added to a solution of 568 mg of naphthol (9) and 768 mg of propargylic alcohol (10) in 10 ml of toluene. The reaction mixture is stirred at 60°C for 4 hours, diluted in dichloromethane and washed with a solution of sodium bicarbonate. After purification by filtration on silica and recrystallisation, 280 mg of crystals are isolated which are pure by ¹H NMR.

EXAMPLE 6: SYNTHESIS OF COMPOUND (6) 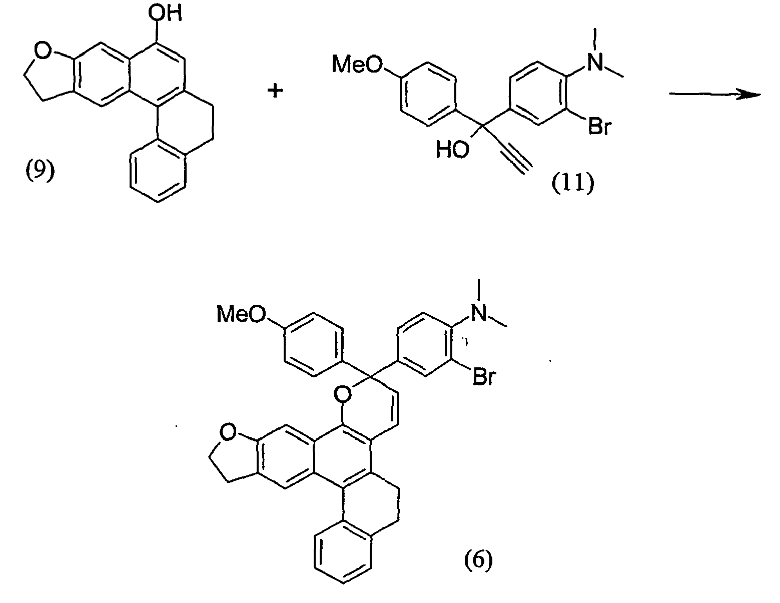 520 mg of camphorsulphonic acid are added to a solution of 580 mg of naphthol (9) and 950 mg propargylic alcohol (11) in 10 ml of toluene. The reaction mixture is stirred at 60°C for 4 hours, diluted in dichloromethane and washed with a solution of sodium bicarbonate. After purification by filtration on silica and recrystallisation, 287 mg of crystals are isolated which are pure by ¹H NMR.

EXAMPLE 7: SYNTHESIS OF COMPOUND (C1) OF PRIOR ART (WO-A-01 36406) 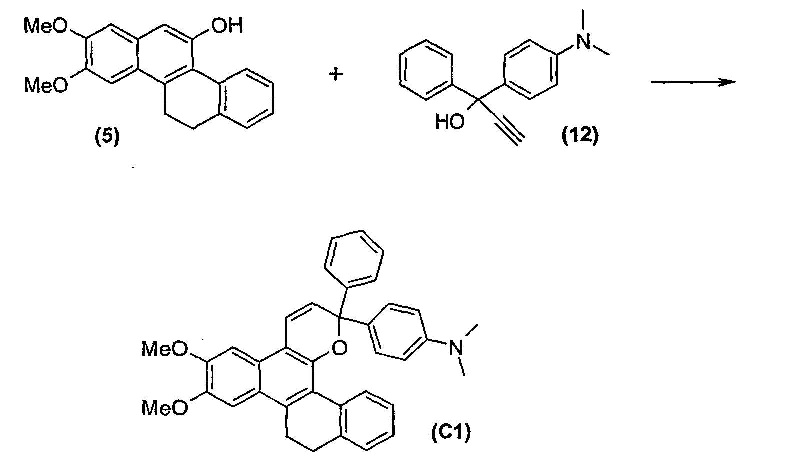 18 mg of camphorsulphonic acid are added to a solution of 460 mg of naphthol (5) and 402 mg of propargylic alcohol (12) in 10 ml of toluene. Stirring is effected at 60°C for 2 hours, and the reaction mixture is then purified by filtration on silica. The solid obtained is recrystallised to give 360 mg of crystals which are pure by ¹H NMR.

EXAMPLE 8: COMPOUND (C2) OF PRIOR ART (WO-A-01 3 6406)

EXAMPLE 9: COMPOUND (C3) OF PRIOR ART (WO-A- 0015628)

### EXAMPLE 10: SYNTHESIS OF COMPOUND (C4) OF PRIOR ART (WO-A-0015628)

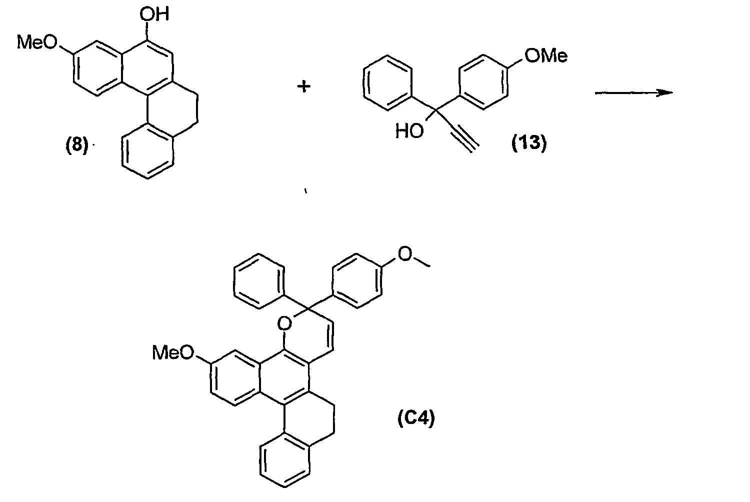

25 mg of camphorsulphonic acid are added to a solution of 553 mg of naphthol (8) and 500 mg of propargylic alcohol (13) in 10 ml of toluene. Stirring is effected at 60°C for 2h, and the reaction mixture is then purified by filtration on silica. The solid obtained is recrystallised to give 640 mg of crystals which are pure by ¹H NMR.

EXAMPLE 11: SYNTHESIS OF COMPOUND (C5) OF PRIOR ART (JP 2001-114775) 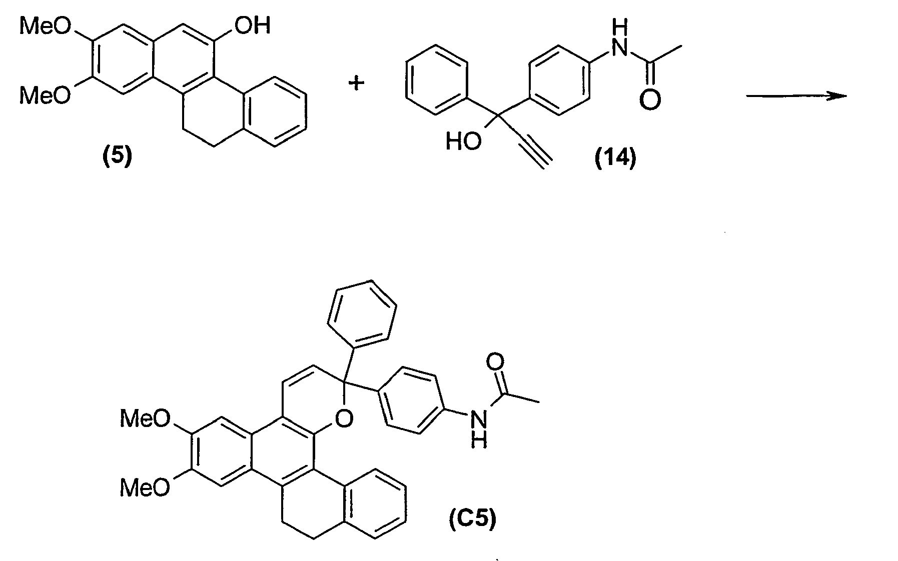 180 mg of camphorsulphonic acid are added to a solution of 500 mg of naphthol (5) and 900 mg propargylic alcohol (14) in 10 ml of toluene. Stirring is effected at 65 °C for 1 hour 30 minutes, and the reaction mixture is then purified by filtration on silica. The solid obtained is recrystallised to give 60 mg of crystals which are pure by ¹H NMR.

The photochromic properties of said compounds (1) to (6) and (C1) to (C5) were evaluated.

Said compounds were dissolved, at the rate of 5 mg in 50 ml of THF, and the UV-visible absorptions (optical path of 1 cm) is then measured before and after exposure to a UV source at 365 nm. The observation of the tints and the intensities developed is done by placing the solutions in the sun or before a solar simulator. The properties of these compounds are given in the Table below.

| COMPOUND | STRUCTURE | λ_{VIS}1* | λ_{MAX} VIS** | T_{1/2}*** |
|---|---|---|---|---|
| (1) | 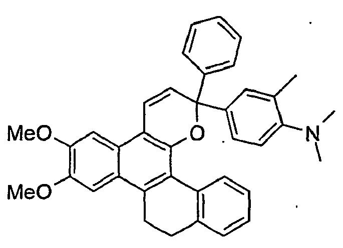 | - | 452 nm | 36 s |
| (2) | 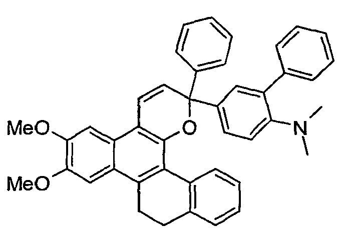 | - | 465 nm | 19 s |
| (3) | 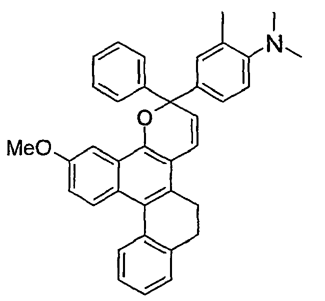 | 435 nm | 556 nm | 69 s |
| (4) | 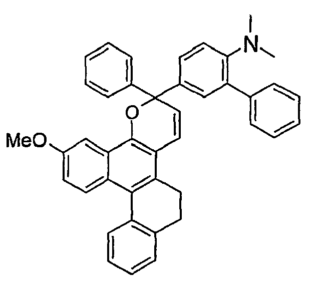 | 469 nm | 562 nm | 61 s |
| (5) | 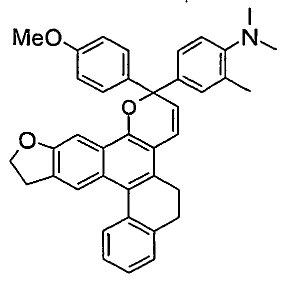 | 444 nm | 563 nm | 37 s |
| (6) | 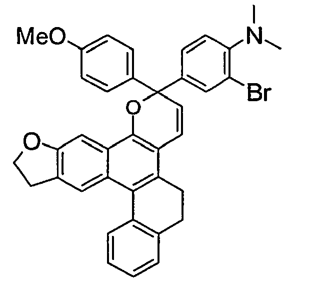 | 443 nm | 561 nm | 43 s |
| (C1) | 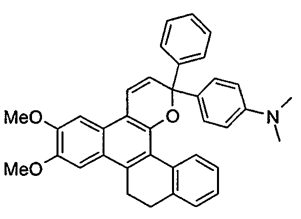 | - | 507 nm | 12 s |
| (C2) | 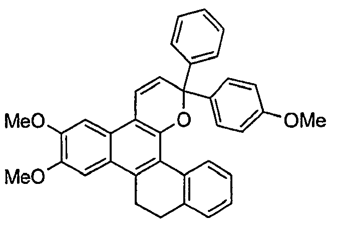 | - | 441 nm | 30 s |
| (C3) | 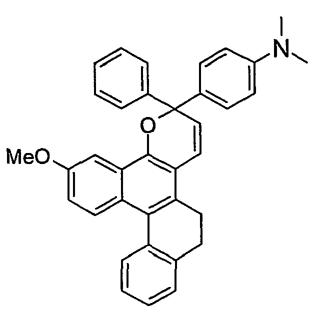 | 482 nm | 584 nm | 20 s |
| (C4) | 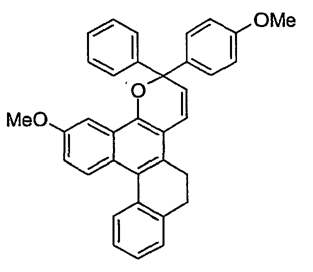 | 429 nm | 548 nm | 55 s |
| (C5) | 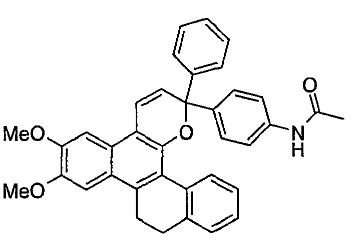 | - | 446 nm | 45 s |

| | | | | |
|---|---|---|---|---|
| • * λ_{VIS}1: λ of the band of highest energy in the field of the visible spectrum of the compound after exposure • ** λ_{MAX} VIS: λ of the band of the lowest energy in the field of the visible spectrum of the compound after exposure. • *** T_{1/2}: discoloration time corresponding to 50% decrease of absorption at the λvis at 21 °C. | | | | |

The observation of the solutions in the presence of sun's rays or UV rays shows that the compounds of the invention have a visible λ_{MAX} between that of the parent compound (C1) or (C3) and that of the alkoxy analogue (C2) or (C4) while at the same time keeping discoloration kinetics close to those of the alkoxy analogue (C2) or (C4). With respect to the N-acyl compounds (C5) of the prior art (JP 2001 114775), the compounds of the invention are unexpectedly much more rapid and their discoloration kinetics are closer to those of the alkoxy analogue (C2).

## Claims

1. Compounds having one or the other of the formulae (Ia), (Ib), (Ic), (Id) below: 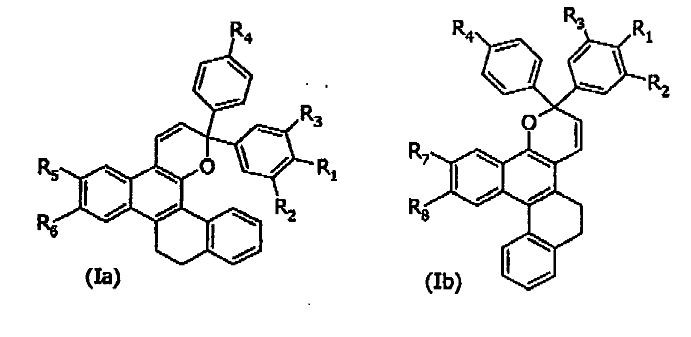 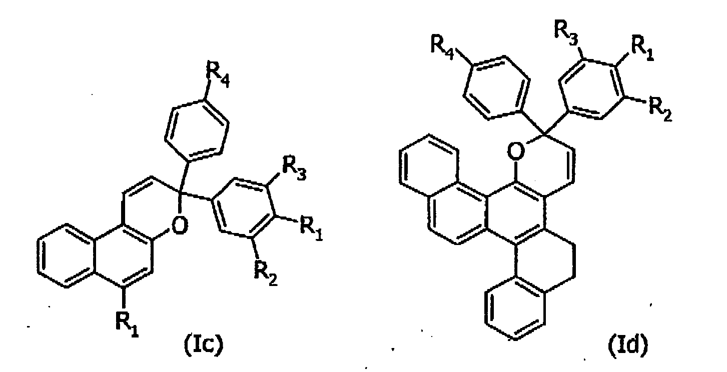 in which:
• R₁ represents a: 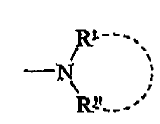 group, R' and R", which are identical or different, independently representing a linear or branched alkyl group which comprises 1 to 6 carbon atoms, a phenyl group which is optionally substituted with at least one linear or branched alkyl group which comprises 1 to 6 carbon atoms, or representing, together with the nitrogen atom to which they are bound, a 5- to 7-membered ring which optionally comprises at least one other heteroatom selected from oxygen, sulphur and nitrogen, said nitrogen being optionally substituted with an R"' group, which is a linear or branched alkyl group having 1 to 6 carbons atoms; the two R₁ substituents of formula (Ic) being, independently, identical or different,
• one of R₂ and R₃ represents:
- chlorine or bromine,
- a linear or branched alkyl group which comprises 1 to 12 carbon atoms (advantageously 1 to 6 carbon atoms),
- a cycloalkyl group which comprises 3 to 12 carbon atoms (advantageously 3 to 6 carbon atoms),
- a linear or branched alkoxy group which comprises 1 to 12 carbon atoms (advantageously 1 to 6 carbon atoms), or
- a phenyl or benzyl group, which is optionally substituted,
and the other of R₂ and R₃ represents:
- a hydrogen, or
- a linear or branched alkyl group which comprises 1 to 6 carbon atoms;
• R₄ represents:
- a hydrogen,
- a linear or branched alkyl group which comprises 1 to 12 carbon atoms (advantageously 1 to 6 carbon atoms),
- a cycloalkyl group which comprises 3 to 12 carbon atoms (advantageously 3 to 6 carbon atoms),
- a linear or branched alkoxy group which comprises 1 to 12 carbon atoms (advantageously 1 to 6 carbon atoms),
- a phenyl or benzyl group, which is optionally substituted;
• R₅ represents: a hydroxy group; a linear or branched alkyl group which comprises 1 to 6 carbon atoms; a linear or branched alkoxy group which comprises 1 to 6 carbon atom; or
an R₁ group, as defined above, *i.e.*, a group having the formula: 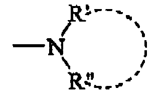
• R₆ represents:
- a halogen, and notably fluorine, chlorine or bromine,
- a hydroxy,
- a linear or branched alkyl group which comprises 1 to 6 carbon atoms,
- a linear or branched alkoxy group which comprises 1 to 6 carbon atoms,
- an aryl or heteroaryl group which comprises, in its basic structure, 6 to 24 carbon atoms or 4 to 24 carbon atoms respectively and at least one heteroatom selected from sulphur, oxygen and nitrogen; said basic structure being optionally substituted with at least one substituent selected from the whole of the substituents given below:
+ a halogen, and notably fluoride, chloride and bromine,
+ a hydroxy,
+ a linear or branched alkyl group which comprises 1 to 12 carbon atoms (advantageously 1 to 6 carbon atoms),
+ a linear or branched alkoxy group which comprises 1 to 12 carbon atoms (advantageously 1 to 6 carbon atoms),
+ a haloalkyl or haloalkoxy group corresponding to the (C₁-C₁₂) alkyl or alkoxy groups above, respectively, which are substituted with at least one halogen atom, and notably a fluoroalkyl group of this type,
+ a phenoxy or naphthoxy group which is optionally substituted with at least one linear or branched alkyl or alkoxy group which comprises 1 to 12 carbon atoms,
+ an -NH₂ group,
+ an -NHR group, R representing a linear or branched alkyl group which comprises 1 to 6 carbon atoms, or a phenyl group which is optionally substituted with at least one linear or branched alkyl group which comprises 1 to 6 carbon atoms,
+ an R₁ group, as defined above, *i.e.,* a group having the following formula: 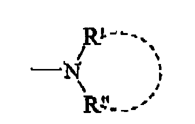 group,
or
R₅ and R₆ together form an aromatic or non-aromatic cyclic group having one or two 5- to 7-membered annelated rings and optionally comprising art least one heteroatom selected from oxygen, sulphur, and nitrogen; said annelated rings optionally comprising at least one R₄ substituent, as defined above, which is different from hydrogen;
• R₇ and R₈, which are identical or different independently represent:
- a hydrogen,
- a halogen, and notably fluorine, chlorine or bromine,
- a linear or branched alkyl group which comprises 1 to 12 carbon atoms (advantageously 1 to 6 carbon atoms),
- a cycloalkyl group which comprises 3 to 12 carbon atoms (advantageously 3 to 6 carbon atoms),
- a linear or branched alkoxy group which comprises 1 to 12 carbon atoms (advantageously 1 to 6 carbon atoms),
- a haloalkyl, halocycloalkyl, or haloalkoxy group corresponding to the alkyl, cycloalkyl or alkoxy groups above, respectively, which are substituted with at least one halogen atom, notably selected from fluorine, chlorine and bromine,
- an aryl or heteroaryl group, having the same definition as the aryl or heteroaryl defined with respect to R₆,
- an aralkyl or heteroaralkyl group, the alkyl group, which is linear or branched, comprising 1 to 4 carbon atoms, and the aryl and heteroaryl groups having the same definitions as those given above,
- a phenoxy or naphtoxy group which is optionally substituted with at least one linear or branched alkyl or alkoxy group which comprises 1 to 12 carbon atoms,
- an amine, amide, carbamate or urea group: -NH₂, -NHR, -NHCOR, NHCOOR, -NR'COR, -NHCONHR, -CONH₂, -CONHR, 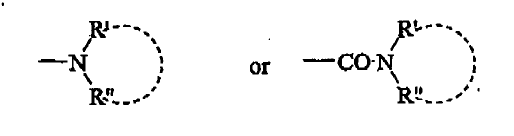 R, R', R" having their respective definitions given above for the amine substituents of R₆ where R₆ is an aryl or heteroaryl,
- an -OCOR₁₁ or -COOR₁₁ a group, R₁₁ representing a straight or branched alkyl group comprising 1 to 6 carbon atoms, or a cycloalkyl group comprising 3 to 6 carbon atoms, or a phenyl group, optionally substituted with at least one substituent selected from the whole of the substituents listed above for R₆ where R₆ is an aryl or heteroaryl,
or
R₇ and R₈ together form an aromatic or non-aromatic cyclic group, optionally comprising at least one heteroatom selected from the group consisting of: oxygen, sulphur, and nitrogen, and having one or two 5- to 7-membered annelated rings; said annelated rings being optionally substituted with at least one substituent selected from the whole of the substituent listed above for R₆ where R₆ is an aryl or heteroaryl.

2. Compounds according to claim 1,
in which:
• one of R₂ and R₃ represents:
chlorine or bromine, a linear or branched alkyl group which comprises 1 to 6 carbon atoms, an optionally substituted phenyl or benzyl group,
and the other of R₂ and R₃ represents:
a hydrogen, or a linear or branched alkyl group which comprises 1 to 6 carbon atoms;
and/or, advantageously and
• R₄ represents:
- a hydrogen,
- a linear or branched alkyl group which comprises 1 to 12 carbon atoms (advantageously 1 to 6 carbon atoms),
- a linear or branched alkoxy group which comprises 1 to 12 carbon atoms (advantageously 1 to 6 carbon atoms).

3. A method of preparing the compounds according to any one of claims 1 to 2, **characterised in that** it comprises carrying out a condensation of:
• an intermediate product of formula (II) given below: 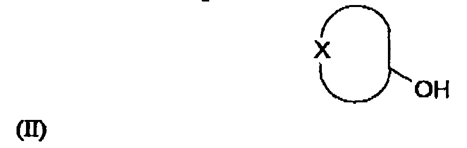 in which the 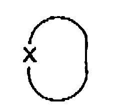 group is one of the following groups 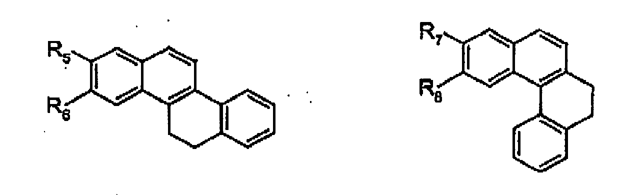 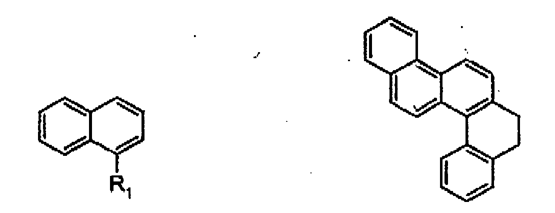 in which
• R₁, R₅, R₆, R₇ and R₈ are as defined above with reference to formulae (Ia) to (Id)
• a derivative of propargylic alcohol, having formula (III) below: 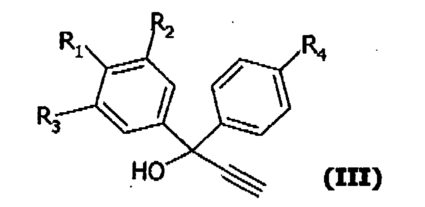 in which R₁, R₂, R₃ and R₄ are as defined in any one of claims 1 to 2;
the condensation of (II) and (III) being carried out advantageously in the presence of a catalyst, this catalyst being preferably selected from the group consisting of para-toluenesulphonic acid, dodecylsulphonic acid, camphorsulphonic acid, or bromoacetic acid;
or with
• an aldehyde derivative, heaving formula (III') below: 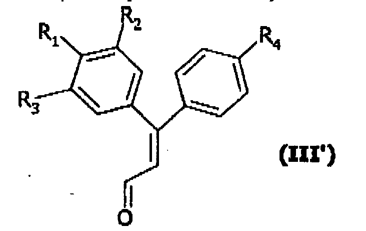 in which R1, R2, R3 and R4 are as defined in any one of claims 1 to 2 the condensation of (II) and (III') being carried out, advantageously, in the presence of a metallic complex, preferably a complex of titanium, particularly titanium (IV) ethoxide.

4. A (co)polymer obtainable by polymerising and/or cross-linking and/or grafting at least one compound according to any one of claims 1 to 2.

5. A photochromic composition, **characterised in that** it is constituted by a compound according to any one of claims 1 to 2, or by a mixture of at least two compounds according to any one of claims 1 to 2, or by a mixture of at least one compound according to one of claims 1 to 2 with at least one other photochromic compound of another type and/or at least one non-photochromic colouring agent.

6. A photochromic composition, **characterised in that** it comprises:
- at least one compound of formula (I) according to any one of claims 1 to 2,
- or at least one (co)polymer according to claim 4,
- and, optionally, at least one other photochromic compound of another type and/or at least one non-photochromic colouring agent and/or at least one stabilising agent.

7. The photochromic composition according to claim 6, **characterised in that** it is selected from the group comprising:
- photochromic solutions, and
- photochromic matrices.

8. The photochromic composition according to one of claims 6 and 7, **characterised in that** it consists of a matrix of (co)polylner(s), the (co)polymer(s) which constitute it being selected from the following list:
- those obtained from alkyl, cycloalkyl, (poly or oligo)ethylene glycol, aryl or arylalkyl mono-, di- tri- or tetraacrylates or mono-, di-, tri- or tetramethacrylates, which is optionally halogenated or comprising at least one ether and/or ester and/or carbonate and/or carbonate and/or thiocarbamate and/or urea and/or amide group,
- polystyrene, polyether, polyester, polycarbonate (*e.g*. bisphenol-A polycarbonate, diallyl diethylene glycol polycarbonate), polycarbamate, polyepoxy, polyurea, polyurethane, polythiourethane, polysiloxane, polyacrylonitrile, polyamide, aliphatic or aromatic polyester, vinylic polymers, cellulose acetate, cellulose triacetate, cellulose acetate-propionate or polyvinylbutyral,
- those obtained from difunctional monomers having the formula below: 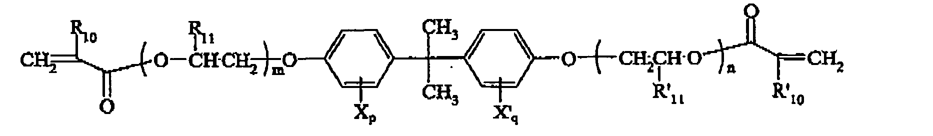 in which:
Δ R₁₀, R'₁₀, R₁₁ and R'₁₁ are identical or different and independently represent a hydrogen or a methyl group;
Δ m and n are, independently, integers between 0 and 4 (inclusive); and are advantageously independently equal to 1 or 2;
Δ X and X', which are identical or different, are a halogen and represent, preferably, a chlorine and/or a bromine;
Δ p and q are, independently, integers between 0 and 4 (inclusive); .
- copolymers of at least two types of copolymerisable monomers selected from the precursor monomers of the polymers listed above, and preferably those belonging to the groups comprising: (meth)acrylic monomers, vinylic monomers, allylic monomers, and mixtures thereof.

9. A photochromic article comprising a photochromic composition according to any one of claims 6 to 8, and more particularly consisting of an ophthalmic or solar lens, a glazing or an optical device.

## Patentansprüche

1. Verbindungen mit einer oder mehreren der nachfolgenden Formeln (Ia), (Ib), (Ic), (Id): 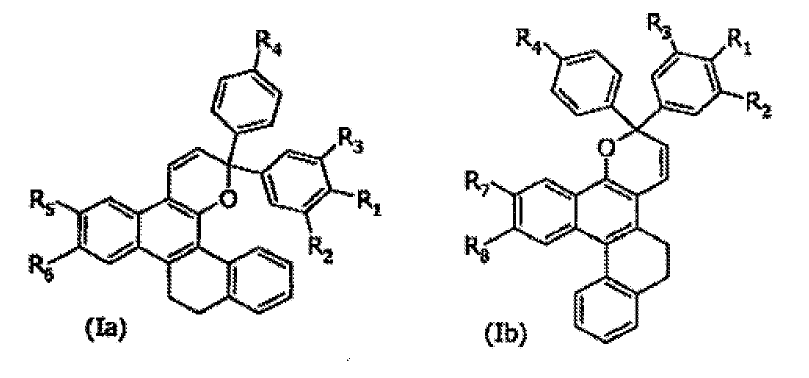 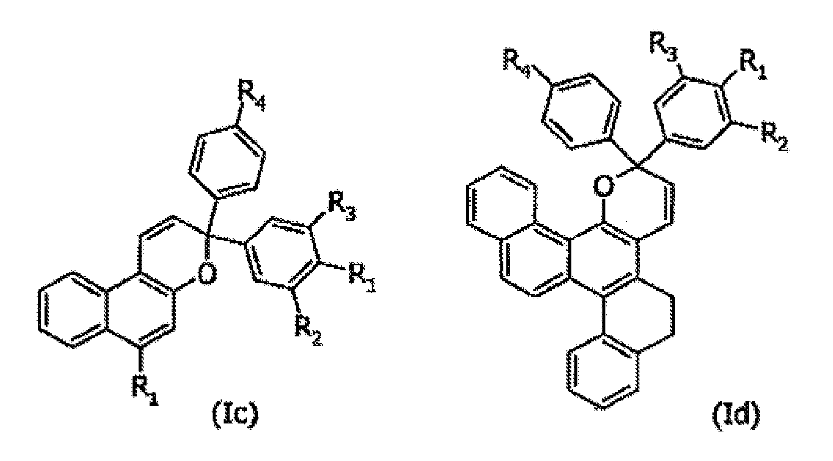 in denen:
• R₁ eine 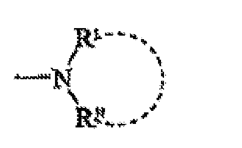 -Gruppe darstellt, R' und R", die gleich oder verschieden sind, unabhängig voneinander eine lineare oder verzweigte Alkylgruppe, die 1 bis 6 Kohlenstoffatome umfasst, eine Phenylgruppe, die optional mit wenigstens einer linearen oder verzweigten Alkylgruppe, die 1 bis 6 Kohlenstoffatome umfasst, substituiert ist, oder zusammen mit dem Stickstoffatom, an das sie gebunden sind, einen 5- bis 7-gliedrigen Ring darstellen, der optional wenigstens ein weiteres Heteroatom umfasst, das ausgewählt ist aus Sauerstoff, Schwefel und Stickstoff, wobei der Stickstoff optional mit einer R'"-Gruppe substituiert ist, die eine lineare oder verzweigte Alkylgruppe ist, die 1 bis 6 Kohlenstoffatome umfasst,
wobei die beiden R₁-Substituenten der Formel (Ic) unabhängig voneinander gleich oder verschieden sind;
• entweder R₂ oder R₃
- Chlor oder Brom,
- eine lineare oder verzweigte Alkylgruppe, die 1 bis 12 Kohlenstoffatome (vorteilhaft 1 bis 6 Kohlenstoffatome) umfasst,
- eine Cycloalkylgruppe, die 3 bis 12 Kohlenstoffatome (vorteilhaft 3 bis 6 Kohlenstoffatome) umfasst,
- eine lineare oder verzweigte Alkoxygruppe, die 1 bis 12 Kohlenstoffatome (vorteilhaft 1 bis 6 Kohlenstoffatome) umfasst, oder
- eine Phenyl- oder Benzylgruppe, die optional substituiert ist,
darstellt
und der andere von R₂ oder R₃
- Wasserstoff oder
- eine lineare oder verzweigte Alkylgruppe, die 1 bis 6 Kohlenstoffatome umfasst,
darstellt;
• R₄
- Wasserstoff,
- eine lineare oder verzweigte Alkylgruppe, die 1 bis 12 Kohlenstoffatome (vorteilhaft 1 bis 6 Kohlenstoffatome) umfasst,
- eine Cycloalkylgruppe, die 3 bis 12 Kohlenstoffatome (vorteilhaft 3 bis 6 Kohlenstoffatome) umfasst,
- eine lineare oder verzweigte Alkoxygruppe, die 1 bis 12 Kohlenstoffatome (vorteilhaft 1 bis 6 Kohlenstoffatome) umfasst,
- eine Phenyl- oder Benzylgruppe, die optional substituiert ist,
darstellt;
• R₅ eine Hydroxygruppe; eine lineare oder verzweigte Alkylgruppe, die 1 bis 6 Kohlenstoffatome umfasst; eine lineare oder verzweigte Alkoxygruppe, die 1 bis 6 Kohlenstoffatome umfasst, oder eine R₁-Gruppe wie sie vorstehend definiert ist, d.h. eine Gruppe mit der Formel 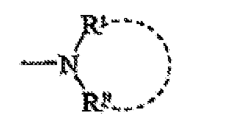 darstellt
• R₆
- ein Halogen und insbesondere Fluor, Chlor oder Brom,
- ein Hydroxy,
- eine lineare oder verzweigte Alkylgruppe, die 1 bis 6 Kohlenstoffatome umfasst,
- eine lineare oder verzweigte Alkoxygruppe, die 1 bis 6 Kohlenstoffatome umfasst,
- eine Aryl- oder Heteroarylgruppe, die als Grundgerüst 6 bis 24 Kohlenstoffatome bzw. 4 bis 24 Kohlenstoffatome und wenigstens ein Heteroatom, das ausgewählt ist aus Schwefel, Sauerstoff und Stickstoff, umfasst,
darstellt,
wobei das Grundgerüst optional mit wenigstens einem Substituenten substituiert ist, der aus den nachfolgend angegebenen Substituenten ausgewählt ist:
+ einem Halogen und insbesondere Fluor, Chlor und Brom,
+ einem Hydroxy,
+ einer linearen oder verzweigten Alkylgruppe, die 1 bis 12 Kohlenstoffatome (vorteilhaft 1 bis 6 Kohlenstoffatome) umfasst,
+ einer linearen oder verzweigten Alkoxygruppe, die 1 bis 12 Kohlenstoffatome (vorteilhaft 1 bis 6 Kohlenstoffatome) umfasst,
+ einer Haloalkyl- oder Haloalkoxygruppe, die den vorstehend genannten (C₁-C₁₂)-Alkyl- bzw. Alkoxygruppen entspricht und mit wenigstens einem Halogenatom und insbesondere einer Fluoralkylgruppe dieses Typs substituiert ist,
+ einer Phenoxy- oder Naphthoxygruppe, die optional mit wenigstens einer linearen oder verzweigten Alkyl- oder Alkoxygruppe substituiert ist, die 1 bis 12 Kohlenstoffatome umfasst,
+ einer -NH₂-Crruppe,
+ einer -NHR-Gruppe, wobei R eine lineare oder verzweigte Alkylgruppe, die 1 bis 6 Kohlenstoffatome umfasst, oder eine Phenylgruppe, die optional mit wenigstens einer linearen oder verzweigten Alkylgruppe substituiert ist, die 1 bis 6 Kohlenstoffatome umfasst, darstellt,
+ einer R₁-Gruppe wie sie vorstehend definiert ist, d.h. eine Gruppe mit der folgenden Formel 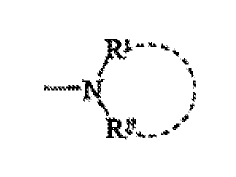
oder
R₅ und R₆ zusammen eine aromatische oder nicht-aromatische cyclische Gruppe, die einen oder zwei 5- bis 7-gliedrige kondensierte Ringe
aufweist und optional wenigstens ein Heteroatom aufweist, das ausgewählt ist aus Sauerstoff, Schwefel und Stickstoff, bilden;
wobei die kondensierten Ringe optional wenigstens einen R₄-Substituenten, wie er vorstehend definiert ist, umfassen, der von Wasserstoff verschieden ist;
• R₇ und R₈, die gleich oder verschieden sind, unabhängig voneinander
- Wasserstoff,
- ein Halogen und insbesondere Fluor, Chlor oder Brom,
- eine lineare oder verzweigte Alkylgruppe, die 1 bis 12 Kohlenstoffatome (vorteilhaft 1 bis 6 Kohlenstoffatome) umfasst,
- eine Cycloalkylgruppe, die 3 bis 12 Kohlenstoffatome (vorteilhaft 3 bis 6 Kohlenstoffatome) umfasst,
- eine lineare oder verzweigte Alkoxygruppe, die 1 bis 12 Kohlenstoffatome (vorteilhaft 1 bis 6 Kohlenstoffatome) umfasst,
- eine Haloalkyl-, Halocycloalkyl- oder Haloalkoxygruppe, die den vorstehend genannten Alkyl-, Cycloalkyl- bzw. Alkoxygruppen entspricht und mit wenigstens einem Halogenatom, das insbesondere ausgewählt ist aus Fluor, Chlor und Brom, substituiert ist,
- eine Aryl- oder Heteroarylgruppe, die in gleicher Weise wie die in Bezug auf R₆ definierte Aryl- oder Heteroarylgruppe definiert ist,
- eine Aralkyl- oder Heteroaralkylgruppe, wobei die Alkylgruppe, die linear oder verzweigt ist, 1 bis 4 Kohlenstoffatome umfasst, und die Aryl- und Heteroarylgruppen in gleicher Weise, wie vorstehend angegeben ist, definiert sind,
- eine Phenoxy- oder Naphthoxygruppe, die optional mit wenigstens einer linearen oder verzweigten Alkyl- oder Alkoxygruppe, die 1 bis 12 Kohlenstoffatome umfasst, substituiert ist,
- eine Amin-, Amid-, Carbamat- oder Harnstoffgruppe: -NH₂, -NHR, - NHCOR, -NHCOOR, -NR'COR, -NHCONHR, -CONH₂, -CONHR, 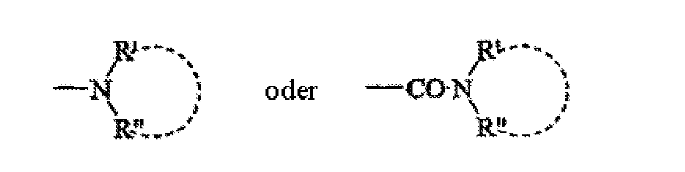 wobei R, R', R" in gleicher Weise, wie vorstehend für die Aminsubstituenten von R₆ angegeben ist, wenn R₆ ein Aryl oder Heteroaryl ist, definiert sind,
- eine -OCOR₁₁- oder -COOR₁₁-Gruppe, wobei R₁₁ eine gerade oder verzweigte Alkylgruppe, die 1 bis 6 Kohlenstoffatome umfasst, oder eine Cycloalkylgruppe, die 3 bis 6 Kohlenstoffatome umfasst, oder eine Phenylgruppe, die optional mit wenigstens einem Substituenten substituiert ist, der ausgewählt ist aus allen vorstehend für R₆ angegebenen Substituenten, wenn R₆ Aryl oder Heteroaryl ist, darstellen,
oder
R₇ und R₈ zusammen eine aromatische oder nicht-aromatische cyclische Gruppe bilden, die optional wenigstens ein Heteroatom umfasst, das ausgewählt ist aus der Gruppe, bestehend aus Sauerstoff, Schwefel und Stickstoff, und einen oder zwei 5- bis 7-gliedrige kondensierte Ringe aufweist;
wobei die kondensierten Ringe optional mit wenigstens einem Substituenten substituiert sind, der ausgewählt ist aus allen vorstehend für R₆ angegebenen Substituenten, wenn R₆ Aryl oder Heteroaryl ist.

2. Verbindungen nach Anspruch 1, in denen:
• R₂ oder R₃
Chlor oder Brom, eine lineare oder verzweigte Alkylgruppe, die 1 bis 6 Kohlenstoffatome umfasst, eine optional substituierte Phenyl- oder Benzylgruppe
darstellt
und der andere von R₂ und R₃
Wasserstoff oder eine lineare oder verzweigte Alkylgruppe, die 1 bis 6 Kohlenstoffatome umfasst,
darstellt;
und/oder, vorteilhaft und,
• R₄
- Wasserstoff,
- eine lineare oder verzweigte Alkylgruppe, die 1 bis 12 Kohlenstoffatome (vorteilhaft 1 bis 6 Kohlenstoffatome) umfasst,
- eine lineare oder verzweigte Alkoxygruppe, die 1 bis 12 Kohlenstoffatome (vorteilhaft 1 bis 6 Kohlenstoffatome) umfasst, darstellt.

3. Verfahren zum Herstellen der Verbindungen gemäß einem der Ansprüche 1 bis 2, **dadurch gekennzeichnet, dass** es das Ausführen einer Kondensation
• eines Zwischenprodukts mit der nachfolgend angegebenen Formel (II): 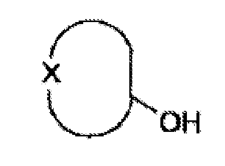 (II), in der die 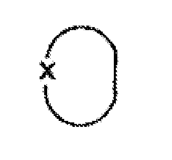
- Gruppe eine der folgenden Gruppen ist: 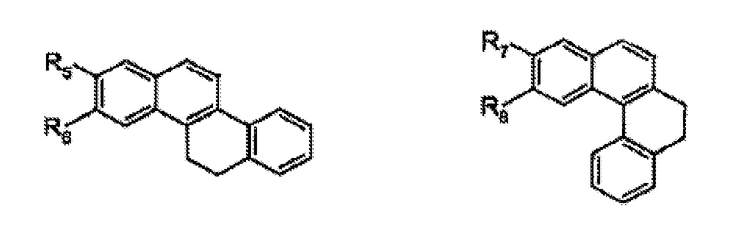 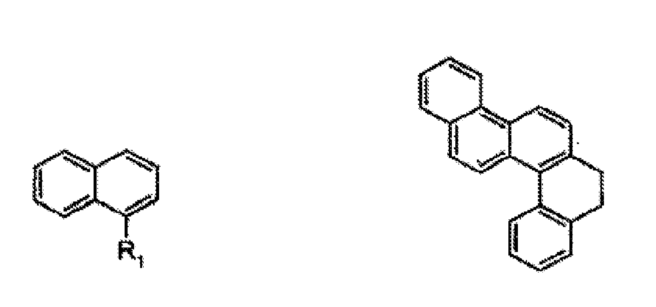 in denen
R₁, R₅, R₆, R₇ und R₈ so sind, wie vorstehend in Bezug auf die Formeln (Ia) bis (Id) definiert sind;
mit
• einem Derivat von Propargylalkohol mit der nachfolgenden Formel III: 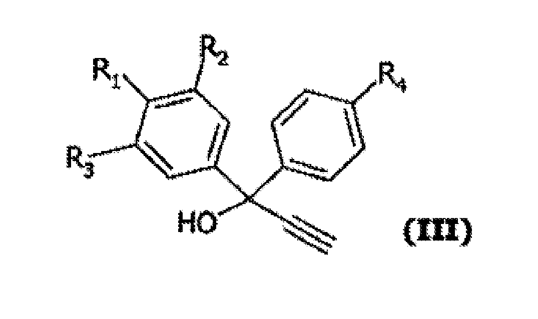 in der R₁, R₂, R₃ und R₄ so sind, wie sie in einem der Ansprüche 1 bis 2 definiert sind;
wobei die Kondensation von (II) und (III) vorteilhaft in Gegenwart eines Katalysators ausgeführt wird und der Katalysator bevorzugt ausgewählt ist aus der Gruppe, bestehend aus para-Toluolsulfonsäure, Dodecylsulfonsäure, Camphersulfonsäure oder Bromessigsäure;
oder mit
einem Aldehydderivat mit der nachfolgenden Formel (III'): 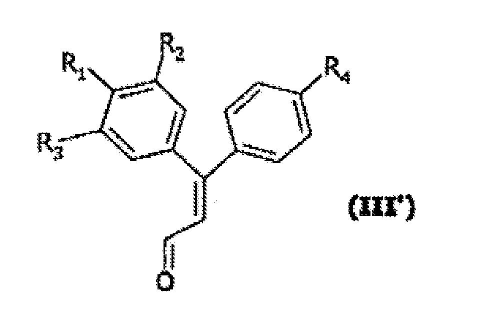 in der R₁, R₂, R₃ und R₄ so sind, wie sie in einem der Ansprüche 1 bis 2 definiert sind,
wobei die Kondensation von (II) und (III') vorteilhaft in Gegenwart eines Metallkomplexes, bevorzugt eines Titankomplexes, insbesondere von Titan(IV)ethoxid, ausgeführt wird,
umfasst.

4. (Co)polymer, erhältlich durch Polymerisieren und/oder Quervemetzen und/oder Pfropfen wenigstens einer Verbindung gemäß einem der Ansprüche 1 bis 2.

5. Photochrome Zusammensetzung, **dadurch gekennzeichnet, dass** sie aus einer Verbindung gemäß einem der Ansprüche 1 bis 2 oder einer Mischung aus wenigstens zwei Verbindungen gemäß einem der Ansprüche 1 bis 2 oder einer Mischung aus wenigstens einer Verbindung gemäß einem der Ansprüche 1 bis 2 und wenigstens einer anderen photochromen Verbindung eines anderen Typs und/oder wenigstens einem nicht-photochromen Farbstoff besteht.

6. Photochrome Zusammensetzung, **dadurch gekennzeichnet, dass** sie umfasst:
- wenigstens eine Verbindung mit der Formel (I) gemäß einem der Ansprüche 1 bis 2,
- oder wenigstens einem (Co)polymer gemäß Anspruch 4,
- und optional wenigstens einer anderen photochromen Verbindung eines anderen Typs und/oder wenigstens einem nicht-photochromen Farbstoff und/oder wenigstens einem Stabilisierungsmittel.

7. Photochrome Zusammensetzung nach Anspruch 6, **dadurch gekennzeichnet**, das sie ausgewählt ist aus der Gruppe, umfassend:
- photochrome Lösungen und
- photochrome Matrizen.

8. Photochrome Zusammensetzung nach einem der Ansprüche 6 und 7, **dadurch gekennzeichnet, dass** sie aus einer Matrix aus (einem) (Co)polymer(en) besteht, wobei das (die) (Co)polymer(e), die diese bilden, ausgewählt sind aus der folgenden Liste:
- solchen, die aus Alkyl, Cycloalkyl, (Poly- oder Oligo-)ethylenglykol, Aryl-oder Arylalkylmono-, -di-, -tri- oder tetraacrylaten oder -mono-, -di-, -tri- oder - tetramethacrylaten, die optional halogeniert sind oder wenigstens eine Ether-und/oder Ester- und/oder Carbonat- und/oder Carbamat- und/oder Thiocarbamat-und/oder Harnstoff- und/oder Amidgruppe umfassen, erhalten wurden,
- Polystyrol, Polyether, Polyester, Polycarbonat (z.B. Bisphenol-A-polycarbonat, Diallyldiethylenglykolpolycarbonat), Polycarbamat, Polyepoxy, Polyharnstoff, Polyurethan, Polythiourethan, Polysiloxan, Polyacrylonitril, Polyamid, aliphatischem oder aromatischem Polyester, Vinylpolymeren, Celluloseacetat, Cellulosetriacetat, Celluloseacetatpropionat oder Polyvinylbutyral,
- solchen, die aus bifunktionalen Monomeren mit der nachfolgenden Formel: 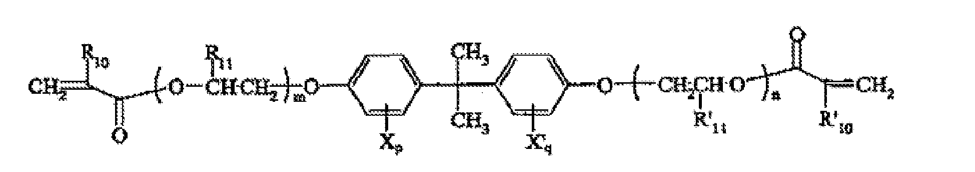 erhalten wurden, in der:
Δ R₁₀, R'₁₀, R₁₁ und R'₁₁ gleich oder verschieden sind und unabhängig voneinander Wasserstoff oder eine Methylgruppe darstellen;
Δ m und n unabhängig voneinander jeweils eine ganze Zahl von 0 bis (einschließlich) 4 sind; und vorteilhaft unabhängig voneinander gleich 1 oder 2 sind;
Δ X und X', die gleich oder verschieden sind, jeweils ein Halogen sind und bevorzugt Chlor und/oder Brom darstellen;
Δ p und q unabhängig voneinander jeweils eine ganze Zahl von 0 bis (einschließlich) 4 sind;
- Copolymeren aus wenigstens zwei Typen copolymerisierbarer Monomere, die ausgewählt sind aus Vorläufermonomeren der vorstehend angegebenen Polymere und bevorzugt solche, die zu den Gruppen gehören, die (Meth)acrylmonomere, Vinylmonomere, Allylmonomere und Mischungen derselben umfassen.

9. Photochromer Gegenstand, umfassend eine photochrome Zusammensetzung gemäß einem der Ansprüche 6 bis 8 und insbesondere bestehend aus einem Brillenglas oder einem Sonnenglas, einer Laser- oder einer optischen Vorrichtung.

## Revendications

1. Composés ayant l'une ou l'autre des formules (Ia), (Ib), (Ic), (Id) ci-dessous : 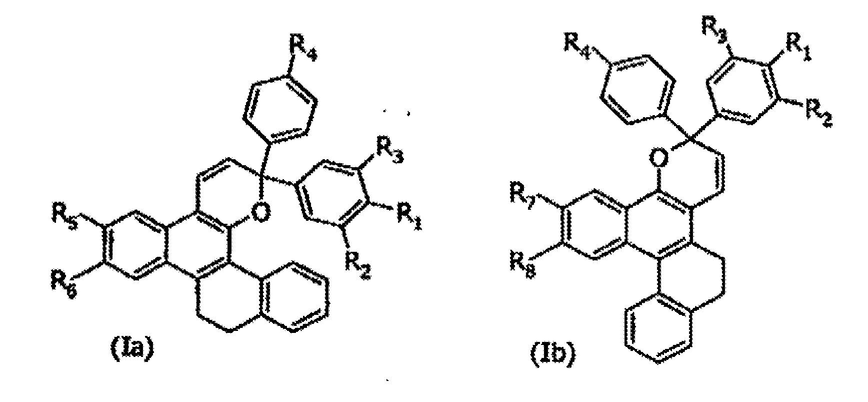 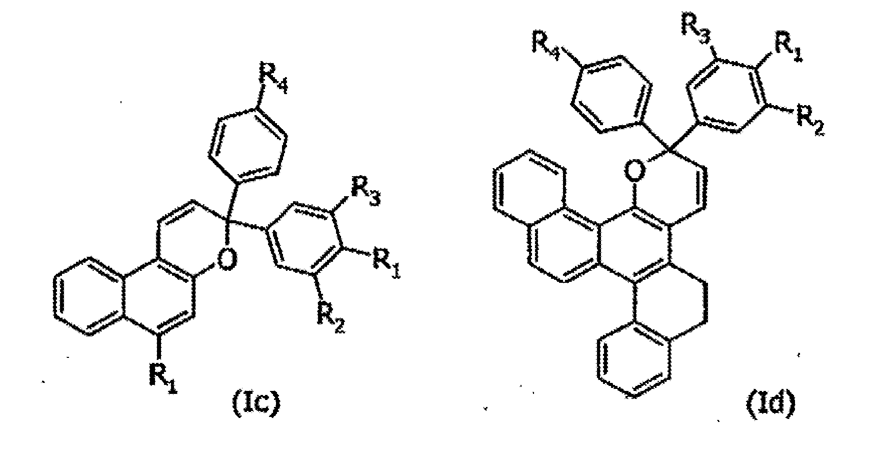 dans lesquelles :
• R₁ représente un groupe : 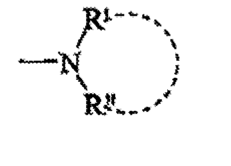 R' et R", qui sont identiques ou différents, représentant de manière indépendante un groupe alkyle linéaire ou ramifié qui comprend 1 à 6 atomes de carbone, un groupe phényle qui est facultativement substitué par au moins un groupe alkyle linéaire ou ramifié qui comprend 1 à 6 atomes de carbone, ou représentant, ensemble avec l'atome d'azote auquel ils sont liés, un cycle de 5 à 7 membres qui comprend facultativement au moins un autre hétéroatome sélectionné parmi l'oxygène, le soufre et l'azote, ledit azote étant facultativement substitué par un groupe R"', qui est un groupe alkyle linéaire ou ramifié ayant 1 à 6 atomes de carbone ; les deux substituants R₁ de la formule (Ic) étant, indépendants, identiques ou différents,
• l'un de R₂ et R₃ représente :
- un atome de chlore ou de brome,
- un groupe alkyle linéaire ou ramifié qui comprend 1 à 12 atomes de carbone (de manière avantageuse 1 à 6 atomes de carbone),
- un groupe cycloalkyle qui comprend 3 à 12 atomes de carbone (de manière avantageuse 3 à 6 atomes de carbone),
- un groupe alcoxy linéaire ou ramifié qui comprend 1 à 12 atomes de carbone (de manière avantageuse 1 à 6 atomes de carbone), ou
- un groupe phényle ou benzyle, qui est facultativement substitué,
et l'autre de R₂ et R₃ représente :
- un atome d'hydrogène, ou
- un groupe alkyle linéaire ou ramifié qui comprend 1 à 6 atomes de carbone ;
• R₄ représente :
- un atome d'hydrogène,
- un groupe alkyle linéaire ou ramifié qui comprend 1 à 12 atomes de carbone (de manière avantageuse 1 à 6 atomes de carbone),
- un groupe cycloalkyle qui comprend 3 à 12 atomes de carbone (de manière avantageuse 3 à 6 atomes de carbone),
- un groupe alcoxy linéaire ou ramifié qui comprend 1 à 12 atomes de carbone (de manière avantageuse 1 à 6 atomes de carbone),
- un groupe phényle ou un benzyle, qui est facultativement substitué ;
• R₅ représente : un groupe hydroxy ; un groupe alkyle linéaire ou ramifié qui comprend 1 à 6 atomes de carbone ; un groupe alcoxy linéaire ou ramifié qui comprend 1 à 6 atomes de carbone ; ou un groupe R₁, tel que défini ci-dessus, c'est-à-dire un groupe ayant la formule : 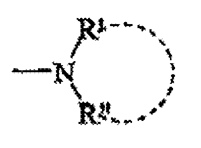
• R₆ représente :
- un atome d'halogène, et notamment de fluor, de chlore ou de brome,
- un groupe hydroxy,
- un groupe alkyle linéaire ou ramifié qui comprend 1 à 6 atomes de carbone,
- un groupe alcoxy linéaire ou ramifié qui comprend 1 à 6 atomes de carbone,
- un groupe aryle ou un groupe hétéroaryle qui comprend, dans sa structure de base, respectivement 6 à 24 atomes de carbone ou 4 à 24 atomes de carbone et au moins un hétéroatome sélectionné parmi le soufre, l'oxygène et l'azote ; ladite structure de base étant facultativement substituée par au moins un substituant sélectionné parmi l'ensemble des substituants indiqués ci-dessous :
+ un atome d'halogène, et notamment de fluor, de chlore et de brome,
+ un groupe hydroxy,
+ un groupe alkyle linéaire ou ramifié qui comprend 1 à 12 atomes de carbone (de manière avantageuse 1 à 6 atomes de carbone),
+ un groupe alcoxy linéaire ou ramifié qui comprend 1 à 12 atomes de carbone (de manière avantageuse 1 à 6 atomes de carbone),
+ un groupe halogénoalkyle ou halogénoalcoxy correspondant, respectivement, aux groupes alkyle ou alcoxy en (C₁-C₁₂) ci-dessus, qui sont substitués par au moins un atome d'halogène, et notamment un groupe fluoroalkyle de ce type,
+ un groupe phénoxy ou naphtoxy qui est facultativement substitué par au moins un groupe alkyle ou alcoxy linéaire ou ramifié qui comprend 1 à 12 atomes de carbone,
+ un groupe -NH₂,
+ un groupe -NHR, R représentant un groupe alkyle linéaire ou ramifié qui comprend 1 à 6 atomes de carbone, ou un groupe phényle qui est facultativement substitué par au moins un groupe alkyle linéaire ou ramifié qui comprend 1 à 6 atomes de carbone,
+ un groupe R₁, tel que défini ci-dessus, c'est-à-dire un groupe ayant la formule suivante : 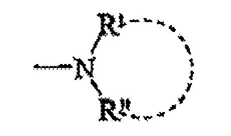 groupe
ou
R₅ et R₆ forment ensemble un groupe cyclique aromatique ou non aromatique ayant un ou deux cycles annelés de 5 à 7 membres et comprenant facultativement au moins un hétéroatome sélectionné parmi l'oxygène, le soufre, et l'azote ; lesdits cycles annelés comprenant facultativement au moins un substituant R₄, tel que défini ci-dessus, qui est différent de l'hydrogène ;
R₇ et R₈, qui sont identiques ou différents, représentent de manière indépendante :
- un atome d'hydrogène,
- un atome d'halogène, et notamment de fluor, de chlore ou de brome,
- un groupe alkyle linéaire ou ramifié qui comprend 1 à 12 atomes de carbone (de manière avantageuse 1 à 6 atomes de carbone),
- un groupe cycloalkyle qui comprend 3 à 12 atomes de carbone (de manière avantageuse 3 à 6 atomes de carbone),
- un groupe alcoxy linéaire ou ramifié qui comprend 1 à 12 atomes de carbone (de manière avantageuse 1 à 6 atomes de carbone),
- un groupe halogénoalkyle, halogénocycloalkyle, ou halogénoalcoxy correspondant, respectivement, aux groupes alkyle, cycloalkyle ou alcoxy ci-dessus qui sont substitués par au moins un atome d'halogène, notamment sélectionné parmi le fluor, le chlore et le brome,
- un groupe aryle ou hétéroaryle, ayant la même définition que le groupe aryle ou hétéroaryle défini par rapport à R₆,
- un groupe aralkyle ou hétéroaralkyle, le groupe alkyle, qui est linéaire ou ramifié, comprenant 1 à 4 atomes de carbone, et les groupes aryle et hétéroaryle ayant les mêmes définitions que celles données ci-dessus,
- un groupe phénoxy ou naphtoxy qui est facultativement substitué par au moins un groupe alkyle ou alcoxy linéaire ou ramifié qui comprend 1 à 12 atomes de carbone,
- un groupe amine, amide, carbamate ou urée : -NH₂, -NHR, - NHCOR, -NHCOOR, -NR'COR, - NHCONHR, -CONH₂, - CONHR, 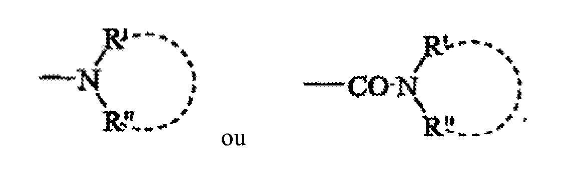 R, R', R" ayant leurs définitions respectives indiquées plus haut pour les substituants amine de R₆ où R₆ représente un groupe aryle ou hétéroaryle,
- un groupe -OCOR₁₁ ou -COOR₁₁, R₁₁ représentant un groupe alkyle linéaire ou ramifié comprenant 1 à 6 atomes de carbone, ou un groupe cycloalkyle comprenant 3 à 6 atomes de carbone, ou un groupe phényle, facultativement substitué par au moins un substituant sélectionné parmi l'ensemble des substituants cités ci-dessus pour R₆ où R₆ représente un groupe aryle ou hétéroaryle,
ou
R₇ et R₈ forment ensemble un groupe cyclique aromatique ou non aromatique, comprenant facultativement au moins un hétéroatome sélectionné parmi le groupe constitué par : l'oxygène, le soufre, et l'azote, et ayant un ou deux cycles annelés de 5 à 7 membres ; lesdits cycles annelés étant facultativement substitués par au moins un substituant sélectionné parmi l'ensemble des substituants cités ci-dessus pour R₆ où R₆ représente un groupe aryle ou hétéroaryle.

2. Composés selon la revendication 1, dans lesquels :
• l'un de R₂ et R₃ représente :
un atome de chlore ou de brome, un groupe alkyle linéaire ou ramifié qui comprend 1 à 6 atomes de carbone, un groupe phényle ou benzyle facultativement substitué, et les autres de R₂ et R₃ représentent:
un atome d'hydrogène, ou un groupe alkyle linéaire ou ramifié qui comprend 1 à 6 atomes de carbone ;
et/ou, de manière avantageuse et
• R₄ représente :
- un atome d'hydrogène
- un groupe alkyle linéaire ou ramifié qui comprend 1 à 12 atomes de carbone (de manière avantageuse 1 à 6 atomes de carbone),
- un groupe alcoxy linéaire ou ramifié qui comprend 1 à 12 atomes de carbone (de manière avantageuse 1 à 6 atomes de carbone).

3. Un procédé de préparation des composés selon l'une quelconque des revendications 1 à 2, **caractérisé en ce qu'**il comprend la mise en oeuvre d'une condensation :
• d'un produit intermédiaire de formule (II) donnée ci-dessous : 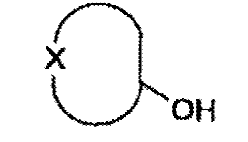 (II)
dans laquelle le groupe 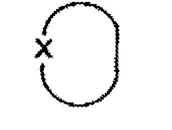 est l'un des groupes suivants : 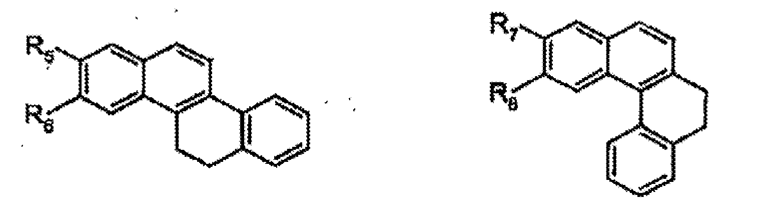 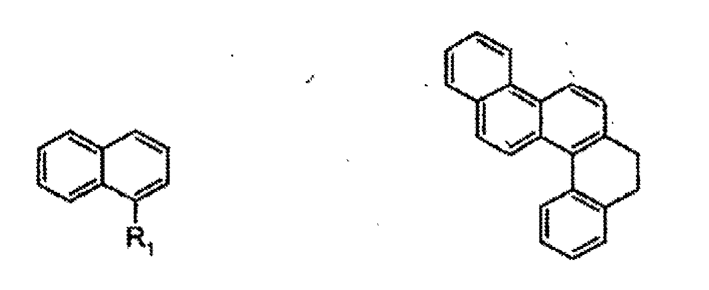 dans lesquels
R₁, R₅, R₆, R₇ et R₈ sont tels que définis ci-dessus en se référant aux formules (la) à (Id),
• d'un dérivé d'alcool propargylique, ayant la formule (III) ci-dessous : 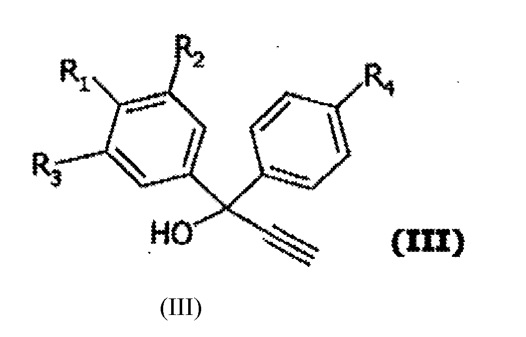 dans laquelle R₁, R₂, R₃ et R₄ sont tels que définis dans l'une quelconque des revendications 1 à 2 ;
la condensation de (II) et (III) étant mise en oeuvre de manière avantageuse en présence d'un catalyseur, ce catalyseur étant, de préférence, sélectionné parmi le groupe constitué par l'acide paratoluènesulfonique, l'acide dodécylsulfonique, l'acide camphorsulfonique, ou l'acide bromoacétique ;
ou avec
• un dérivé aldéhydique, ayant la formule (III') ci-dessous : 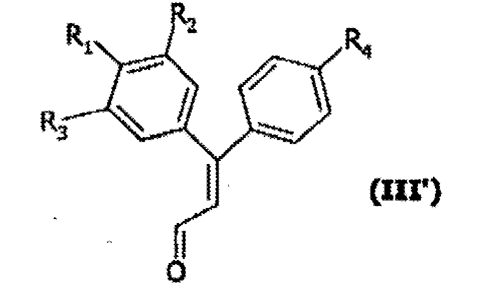 dans laquelle R₁, R₂, R₃ et R₄ sont tels que définis dans l'une quelconque des revendications 1 à 2 ;
la condensation de (II) et (III') étant effectuée, de manière avantageuse, en présence d'un complexe métallique, de préférence un complexe de titane, en particulier d'éthoxyde de titane (IV).

4. Un (co)polymère pouvant être obtenu par polymérisation et/ou réticulation et/ou greffe d'au moins un composé selon l'une quelconque des revendications 1 à 2.

5. Une composition photochromique, **caractérisée en ce qu'**elle est constituée d'un composé selon l'une quelconque des revendications 1 à 2 ; ou d'un mélange d'au moins deux composés selon l'une quelconque des revendications 1 à 2, ou bien d'un mélange d'au moins un composé selon l'une des revendications 1 à 2 avec au moins un autre composé photochromique d'un autre type et/ou au moins un agent colorant non photochromique.

6. Une composition photochromique, **caractérisée en ce qu'**elle comprend :
- au moins un composé de formule (I) selon l'une quelconque des revendications 1 à 2,
- ou au moins un (co)polymère selon la revendication 4,
- et, facultativement, au moins un autre composé photochromique d'un autre type et/ou au moins un agent colorant non photochromique et/ou au moins un agent stabilisant.

7. La composition photochromique selon la revendication 6, **caractérisée en ce qu'**elle est sélectionnée parmi le groupe comprenant :
- des solutions photochromiques, et
- des matrices photochromiques.

8. La composition photochromique selon l'une des revendications 6 et 7, **caractérisée en ce qu'**elle consiste en une matrice de (co)polymère(s), les (co)polymère(s) qui la constituent étant sélectionnés parmi la liste suivante :
- ceux qui sont obtenus à partir de mono-, di- tri- ou tétraacrylates ou mono-, di-, tri- ou tétraméthacrylates d'alkyle, de cycloalkyle, de (poly ou oligo)éthylène glycol, d'aryle ou d'arylalkyle, qui sont facultativement halogénés ou comprenant au moins un groupe éther et/ou ester et/ou carbonate et/ou carbamate et/ou thiocarbamate et/ou urée et/ou amide,
- un polystyrène, un polyéther, un polyester, un polycarbonate (par exemple le polycarbonate de bisphénol-A, le polycarbonate de diallyle/diéthylène glycol), un polycarbamate, un polyépoxy, une polyurée, un polyuréthane, un polythiouréthane, un polysiloxane, un polyacrylonitrile, un polyamide, un polyester aliphatique ou aromatique, des polymères vinyliques, l'acétate de cellulose, le triacétate de cellulose, l'acétate-propionate de cellulose ou le polyvinylbutyral,
- ceux qui sont obtenus à partir de monomères bifonctionnels ayant la formule ci-dessous : 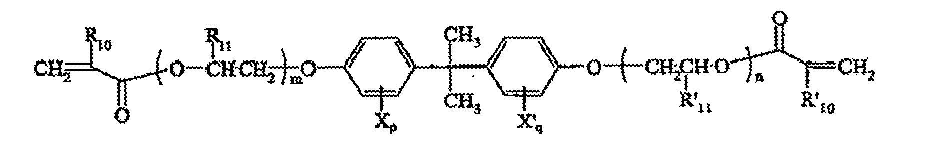 dans laquelle :
Δ R₁₀, R'₁₀, R₁₁ et R'₁₁ sont identiques ou différents et représentent de manière indépendante un atome d'hydrogène ou un groupe méthyle ;
Δ m et n sont, de manière indépendante, des nombres entiers entre 0 et 4 (inclus) ;
et sont, de manière avantageuse, égaux à 1 ou 2, de manière indépendante ;
Δ X et X', qui sont identiques ou différents, représentent un atome d'halogène et représentent, de préférence, un atome de chlore et/ou de brome ;
Δ p et q sont, de manière indépendante, des nombres entiers entre 0 et 4 (inclus) ;
- des copolymères d'au moins deux types de monomères copolymérisables sélectionnés parmi les monomères précurseurs des polymères listés ci-dessus et, de préférence, ceux qui appartiennent aux groupes comprenant : les monomères (méth)acryliques, les monomères vinyliques, les monomères allyliques, et des mélanges de ceux-ci.

9. Un article photochromique comprenant une composition photochromique selon l'une quelconque des revendications 6 à 8, et consistant plus particulièrement en une lentille ophtalmique ou solaire, un dispositif pour glaçure ou un dispositif optique.
